# EUROPEAN PATENT SPECIFICATION

(11) **EP 4 160 210 B1**
(45) Date of publication and mention of the grant of the patent: **18.12.2024**
(21) Application number: 22204041.2
(22) Date of filing: 25.06.2018
(51) Int. Cl.: G01N 33/543, G01N 33/558, G01N 21/64, G01N 21/84

(54) **SANDWICH-TYPE ASSAYS USING DECREASING SIGNAL PORTIONS OF DOSE RESPONSE CURVE TO MEASURE ANALYTES, INCLUDING ANALYTES AT HIGH CONCENTRATION**
SANDWICH-ASSAYS MIT ABNEHMENDEN SIGNALTEILEN EINER DOSISREAKTIONSKURVE ZUR MESSUNG VON ANALYTEN, EINSCHLIESSLICH ANALYTEN IN HOHER KONZENTRATION
DOSAGES DE TYPE SANDWICH UTILISANT DES PARTIES DE SIGNAL DE DIMINUTION DE COURBE DE RÉPONSE DE DOSE POUR MESURER DES ANALYTES, Y COMPRIS DES ANALYTES À CONCENTRATION ÉLEVÉE

(30) Priority: 28.06.2017 US 201762526051 P
(43) Date of publication of application: 05.04.2023
(62) Divisional of application: 18825104.5
(73) Proprietor: Becton, Dickinson and Company, Franklin Lakes, NJ 07417-1880 (US)
(72) Inventor: YANG, Jian, Franklin Lakes, NJ, 07417 (US); REN, Huimiao, Franklin Lakes, NJ, 07417 (US)
(74) Representative: Vossius & Partner Patentanwälte Rechtsanwälte mbB

(56) References cited:
- EP-A2- 0 987 551
- WO-A1-2013/088429
- WO-A2-2013/132338
- US-A1- 2009 180 927
- US-A1- 2009 246 886
- US-A1- 2009 253 219
- US-B1- 6 924 153
- US-B2- 7 439 079

## Description

### FIELD

The present disclosure relates in general to lateral flow assay devices, test systems, and methods. More particularly, the present disclosure relates to lateral flow assay devices to determine the concentration of analyte in a sample, including when the analyte of interest is present at high concentrations.

### BACKGROUND

Immunoassay systems, including lateral flow assays described herein provide reliable, inexpensive, portable, rapid, and simple diagnostic tests. Lateral flow assays can quickly and accurately detect the presence or absence of, and in some cases quantify, an analyst of interest in a sample. Advantageously, lateral flow assays can be minimally invasive and used as point-of-care testing systems. Lateral flow assays have been developed to detect a wide variety of medical or environmental analytes. In a sandwich format lateral flow assay, a labeled antibody against an analyte of interest is deposited on a test strip in or near a sample receiving zone. The labeled antibody may include, for example, a detector molecule or "label" bound to the antibody. When the sample is applied to the test strip, analyte present in the sample is bound by the labeled antibody, which flows along the test strip to a capture zone, where an immobilized antibody against the analyte binds the labeled antibody-analyte complex. The antibody immobilized on the capture line may be different than the labeled antibody deposited in or near the sample receiving zone. The captured complex is detected, and the presence of analyte is determined. In the absence of analyte, the labeled antibody flows along the test strip but passes by the capture zone. The lack of signal at the capture zone indicates the absence of analyte. The sandwich lateral flow assay, however, suffers from many disadvantages, including false negatives, inaccurately low results, and lack of resolution when the analyte of interest is present in the sample at high concentrations. US 6 924 153, US 7 439 079, EP 0 987 551, US 2009/180927, WO 2013/132338, WO 2013/088429, US 2009/253219 and US 2009/246886 are documents of the related art.

### SUMMARY

It is therefore an aspect of this disclosure to provide improved lateral flow assays that precisely measure the concentration of an analyte of interest in a sample, including when the analyte is present in the sample at high concentrations.
The invention is defined in the independent claims. Dependent claims describe preferred embodiments.

Some examples disclosed herein relate to an assay test strip including a flow path configured to receive a fluid sample; a sample receiving zone coupled to the flow path; a capture zone; and a complex. The capture zone is coupled to the flow path downstream of the sample receiving zone and includes an immobilized capture agent specific to the analyte of interest. The complex is coupled to the flow path in a first phase and configured to flow in the flow path to the capture zone in the presence of the fluid sample in a second phase. The complex includes a label, an antibody or a fragment of an antibody that specifically binds the analyte of interest, and the analyte of interest. In some cases, the flow path is configured to receive a fluid sample comprising unlabeled analyte of interest, and the complex does not specifically bind to the unlabeled analyte of interest in the first phase or the second phase. In some instances, the complex is configured to flow with the unlabeled analyte of interest in the flow path to the capture zone in the second phase. In some examples, the complex is configured to compete with the unlabeled analyst of interest to bind to the immobilized capture agent in the capture zone in a third phase. In some cases, an optical signal emitted from complex bound to the immobilized capture agent in the capture zone decreases as concentration of unlabeled analyte of interest in the fluid sample increases.

In some examples, the flow path is configured to receive a fluid sample that does or does not include analyte of interest. The complex specifically binds to all or substantially all of the immobilized capture agent in the capture zone in the second phase when the fluid sample does not include analyte of interest. In some instances, when the fluid sample does not include analyte of interest, an optical signal emitted from the complex bound in the capture zone is a maximum optical signal that can be emitted from the assay test strip. When the fluid sample does include analyte of interest, an optical signal emitted from the complex bound in the capture zone is less than the maximum optical signal.

In some cases, the immobilized capture agent includes an antibody or a fragment of an antibody that specifically binds the analyte of interest. The complex is integrated onto a surface of the test strip in a first phase in some examples. In some instances, the complex is integrated onto the surface of the test strip by spraying a solution comprising the complex onto the surface of the test strip and drying the solution. The fluid sample can include a blood, plasma, urine, sweat, or saliva sample. In one non-limiting example, the analyte of interest includes C-reactive protein (CRP) and the complex includes an anti-CRP antibody or fragment thereof bound to the CRP.

Other examples disclosed herein relate to a diagnostic test system including an assay test strip described above; a reader including a light source and a detector, and a data analyzer. In some cases, the data analyzer outputs an indication that there is no analyte of interest in the fluid sample when the reader detects an optical signal from the assay test strip that is a maximum optical signal of a dose response curve of the test strip. In one example, the data analyzer outputs an indication that there is a low concentration of analyte of interest in the fluid sample when the reader detects an optical signal from the assay test strip that is within 1 % of the maximum optical signal. In another example, the data analyzer outputs an indication that there is a low concentration of analyte of interest in the fluid sample when the reader detects an optical signal from the assay test strip that is within 5% of the maximum optical signal. In still another example, the data analyzer outputs an indication that there is a low concentration of analyte of interest in the fluid sample when the reader detects an optical signal from the assay test strip that is within 10% of the maximum optical signal. In a further example, the data analyzer outputs an indication that there is a high concentration of analyte of interest in the fluid sample when the reader detects an optical signal from the assay test strip that is 90% or less than 90% of the maximum optical signal. In yet another example, the data analyzer outputs an indication of the concentration of analyte of interest in the sample when the reader detects an optical signal from the assay test strip that is below the maximum optical signal.

An embodiment disclosed herein relates to a method of measuring an analyte of interest present in a fluid sample. The method includes applying the fluid sample to an assay test strip described above when the complex is coupled to the flow path in the first phase; uncoupling the complex from the flow path; flowing the fluid sample and the complex in the flow path to the capture zone in the second phase; binding the complex to the immobilized capture agent in the capture zone; and detecting a signal from the complex bound to the immobilized capture agent in the capture zone. The detected signal can be an optical signal, a fluorescence signal, or a magnetic signal. In some cases, uncoupling the complex includes solubilizing the complex with the fluid sample. In some instances, the fluid sample includes unlabeled analyte of interest, and the complex does not specifically bind to the unlabeled analyte of interest in the first phase or the second phase. In another instance, the fluid sample includes unlabeled analyte of interest, and the complex is configured to compete with the unlabeled analyst of interest to bind to the immobilized capture agent in the capture zone in the third phase. In one example, the fluid sample does not include analyte of interest, and detecting includes detecting a maximum optical signal of a dose response curve of the test strip.

In some cases, the method includes determining that the concentration of analyte in the fluid sample is zero. In some instances, the method further includes displaying an indication that the analyte of interest is not present in the fluid sample.

In one example, the fluid sample includes analyte of interest, and detecting includes detecting a signal from the test strip that is less than a maximum signal of a dose response curve of the test strip. In some cases, the method further includes determining that the concentration of analyte in the fluid sample is greater than zero. In some instances, the method further includes displaying an indication that the analyte of interest is present in the fluid sample. In one example, the method further includes determining that the detected signal is within 10% of the maximum optical signal; and displaying an indication that the analyte of interest is present in the fluid sample at low concentration. In another example, the method further includes determining that the detected signal is 90% or less than 90% of the maximum signal; and displaying an indication that the analyte of interest is present in the fluid sample at high concentration.

Additional embodiments disclosed herein relate to a method of manufacturing an assay test strip including coupling a sample receiving zone to a flow path configured to receive a fluid sample; coupling a capture zone to the flow path downstream of the sample receiving zone; and coupling a complex to the flow path. The complex includes a label; an antibody or a fragment of an antibody that specifically binds an analyte of interest; and the analyte of interest. In some cases, the analyte of interest includes C-reactive protein (CRP) and the antibody includes anti-CRP antibody or a fragment of anti-CRP antibody. In one instance, the analyte of interest includes about 50 ng of CRP. In another instance, the analyte of interest includes about 100 ng of CRP. In some cases, the method further includes immobilizing a capture agent specific to the analyte of interest on the capture zone. In some instances, coupling the complex to the flow path includes forming a bond between the complex and the flow path that breaks in the presence of fluid sample in the flow path. In one example, coupling the complex includes spraying a solution including the complex onto a surface of the sample receiving zone. In another example, coupling the complex includes spraying a solution including the complex onto a surface of the assay test strip between the sample receiving zone and the capture zone. In a further example, coupling the complex includes applying a fluid solution including the complex onto a surface of the assay test strip; and drying the fluid solution. In still another example, coupling the complex includes integrating the complex into a surface of the assay test strip.

In some instances, the method further includes providing a solution including the complex. In some cases, providing the solution includes mixing a first liquid including the label and the antibody or fragment of the antibody with a second liquid including the analyte of interest. In some examples, providing the solution further includes incubating the mixture of the first liquid and the second liquid for about 30 minutes. In some instances, coupling the complex to the flow path includes spraying the solution onto a surface of the assay test strip. Still further embodiments disclosed herein relate to assay test strips made by the methods described above.

### BRIEF DESCRIPTION OF THE DRAWINGS

Figures 1A and 1B illustrate an example sandwich-type lateral flow assay before and after a fluid sample is applied at a sample receiving zone.
Figure 2 illustrates an example dose response curve for the lateral flow assay of Figures 1A and 1B.
Figures 3A and 3B illustrate an example competitive-type lateral flow assay before and after a fluid sample is applied at a sample receiving zone.
Figure 4 illustrates an example dose response curve for a competitive lateral flow assay of Figures 3A and 3B.
Figures 5A and 5B illustrate an example lateral flow assay according to the present disclosure before and after a fluid sample is applied at a sample receiving zone.
Figure 5C illustrates an example dose response curve for the lateral flow assay of Figures 5A and 5B.
Figure 6A illustrates an example dose response curve for a sandwich-type lateral flow assay such as that illustrated in Figures 1A and 1B and an example dose response curve for a lateral flow assay according to the present disclosure, where concentration of analyte is measured along the x-axis in logarithmic scale.
Figure 6B illustrates the example dose response curve of Figure 6A for a lateral flow assay according to the present disclosure where the concentration of analyte is measured along the x-axis in non-logarithmic scale.
Figures 7A and 7B illustrate a table of experimental data and a graph representing the experimental data, respectively, that correlate the concentration of CRP as measured by a lateral flow assay according to according to one embodiment of the present disclosure with the concentration of CRP as determined by ELISA.

### DETAILED DESCRIPTION

Devices, systems and methods described herein precisely determine the quantity of an analyte of interest in a sample, for example a concentration of the analyte in a sample of known volume. Advantageously, lateral flow devices, test systems, and methods according to the present disclosure precisely determine the quantity of an analyte of interest in situations where the analyte of interest is present in the sample at an elevated or "high" concentration. Lateral flow assays described herein can generate a signal of maximum intensity when the concentration of analyte of interest in the sample is zero. Signals generated by assays according to the present disclosure are described herein in the context of an optical signal generated by reflectance-type labels (such as but not limited to gold nanoparticle labels). Although embodiments of the present disclosure are described herein by reference to an "optical" signal, it will be understood that assays described herein can use any appropriate material for a label in order to generate a detectable signal, including but not limited to fluorescence-type latex bead labels that generate fluorescence signals and magnetic nanoparticle labels that generate signals indicating a change in magnetic fields associated with the assay. For low concentrations of analyte, the lateral flow assays described herein generate optical signals that are the same as or substantially equivalent to (within a limited range of variance from) the maximum intensity signal. Lateral flow assays according to the present disclosure generate signals that are less than the maximum intensity signal for elevated or "high" concentrations of analyte of interest.

According to the present disclosure, a labeled agent including a label-antibody-analyte complex is initially integrated onto a surface, for example onto the conjugate pad, of a lateral flow assay test strip. The label-antibody-analyte complex becomes unbound from the label zone upon application of a fluid sample to the test strip, and travels to the capture zone of the test strip with the fluid sample and any analyte of interest in the sample (if present). The label-antibody-analyte complex and analyte of interest in the sample (when present) bind to capture agent in the capture zone. The capture agent binds completely to the label-antibody-analyte complex when there is no analyte of interest in the sample to compete with the label-antibody-analyte complex, generating a signal of maximum intensity. When analyte of interest is present in the sample in low concentrations, the label-antibody-analyte complex competes with a relatively low amount of unlabeled analyte to bind to capture agent, resulting in a signal that is the same as or substantially equivalent to (within a limited range of variance from) the maximum intensity signal. When analyte of interest is present in the sample in high concentrations, the label-antibody-analyte complex competes with a relatively high amount of unlabeled analyte to bind to capture agent, resulting in a signal that is less than the maximum intensity signal.

Without being bound to any particular theory, the addition of labeled analyte in the form of the label-antibody-analyte complex integrated in the label zone masks the portion of a sandwich-type lateral flow assay dose response curve where signals are increasing (when analyte concentrations are low), thereby generating an improved dose response curve that starts at a maximum intensity signal at zero concentration and then either remains relatively constant (analyte at low concentrations) or decreases (analyte at high concentrations). Lateral flow assays of the present disclosure solve drawbacks associated with the hook effect of sandwich-type lateral flow assays by eliminating the phase of the dose response curve where signals are increasing.

Signals generated by lateral flow assays described herein when the analyte is at high concentrations include many advantageous features. In example embodiments that generate optical signals, signals that are generated when the analyte is at high concentration are readily detectable (for example, they have an intensity within a range of optical signals which conventional readers can typically discern and are well spaced apart), they do not overlap on the dose response curve with signals generated at zero or low concentrations, and they can be used to calculate a highly-accurate concentration reading at high and even very high concentrations. Embodiments of the lateral flow assays described herein avoid uncertainty associated with correlating a particular detected signal with a quantity of analyte (especially analyte at high concentration), such as uncertainty that occurs in reading sandwich-type lateral flow assays that generate a single optical signal corresponding to both a low concentration and a high concentration of analyte due to the hook effect. In contrast, lateral flow assays according to the present disclosure generate an optical signal that clearly and unambiguously corresponds to a zero or low concentration of analyte (optical signal at or substantially equivalent to the maximum intensity signal) or a high concentration of analyte (optical signal less than the maximum intensity signal). In some cases, zero or low concentrations can be directly correlated to a normal or "healthy" level of analyte in the subject, and high concentrations of analyte can be directly correlated to a non-normal or "unhealthy" level of analyte in the subject.

Furthermore, embodiments of the lateral flow assay according to the present disclosure strongly correlate with current gold standard assays for determining the quantity of analyte in a sample, such as enzyme-linked immunosorbent assay (ELISA). Advantageously, the concentration of CRP as determined by embodiments of the lateral flow assays described herein has been discovered to strongly correlate with the concentration of CRP as determined by ELISA. In one working example described below, a correlation of 93% between concentration of CRP measured using an embodiment of assays according to the present disclosure and concentration of CRP determined by ELISA was obtained.

Embodiments of the lateral flow assay described herein are particularly advantageous in diagnostic tests for analytes of interest that naturally occur at low concentrations in healthy individuals but elevate to high concentrations in individuals with a disease condition or disorder. Optical signals with relatively little variance from a maximum intensity signal are generated in the zero to low concentration range where the operator only seeks to confirm that the analyte is present at a low concentration (indicator of healthy levels) and does not require specificity or resolution of optical signals, while readily-detectable, high resolution optical signals with high variance from the maximum intensity signal are generated where the operator seeks to confirm that the analyte is present at high concentration (indicator of a not-normal or disease condition) and in particular seeks to quantify the analyte of interest whenever it is at high concentrations. The ability to accurately pinpoint the precise concentration of an analyte of interest when it is within a range of high concentrations can also allow the operator to ascertain the stage or progress of a disease or other condition in the subject, such as a mild stage or a severe stage.

Various aspects of the lateral flow assays provide advantages over existing lateral flow assays. For example, in some embodiments, the lateral flow assays described herein do not require multiple test lines, but instead, have the ability to both accurately determine the concentration of an analyte and also determine whether the test functioned properly with the use of only one capture line. Furthermore, in some embodiments, the lateral flow assays described herein can accurately determine the concentration of elevated analyte in a sample without the requirement to first dilute the sample. In addition, in some embodiments, the amount of pre-formed label-antibody-analyte complex placed on the lateral flow assay can be varied to accommodate the requirement of different concentration ranges of analytes.

Various aspects of the devices, test systems, and methods are described more fully hereinafter with reference to the accompanying drawings. The disclosure may, however, be embodied in many different forms. Based on the teachings herein one skilled in the art should appreciate that the scope of the disclosure is intended to cover any aspect of the devices, test systems, and methods disclosed herein, whether implemented independently of or combined with any other aspect of the present disclosure. For example, a device may be implemented or a method may be practiced using any number of the aspects set forth herein.

Although particular aspects are described herein, many variations and permutations of these aspects fall within the scope of the disclosure. Although some benefits and advantages are mentioned, the scope of the disclosure is not intended to be limited to particular benefits, uses, or objectives. Rather, aspects of the disclosure are intended to be broadly applicable to different detection technologies and device configurations some of which are illustrated by way of example in the figures and in the following description. The detailed description and drawings are merely illustrative of the disclosure rather than limiting, the scope of the disclosure being defined by the appended claims and equivalents thereof.

Lateral flow devices described herein are analytical devices used in lateral flow chromatography. Lateral flow assays are assays that can be performed on lateral flow devices described herein. Lateral flow devices may be implemented on a test strip but other forms may be suitable. In the test strip format, a test sample fluid, suspected of containing an analyte, flows (for example by capillary action) through the strip. The strip may be made of bibulous materials such as paper, nitrocellulose, and cellulose. The sample fluid is received at a sample reservoir. The sample fluid can flow along the strip to a capture zone in which the analyte (if present) interacts with a capture agent to indicate a presence, absence, and/or quantity of the analyte. The capture agent can include antibody immobilized in the capture zone.

### Sandwich-type and Competitive-Type Lateral Flow Assays

Lateral flow assays can be performed in a sandwich or competitive format. Sandwich and competitive format assays described herein will be described in the context of reflective-type labels (such as gold nanoparticle labels) generating an optical signal, but it will be understood that assays may include latex bead labels configured to generate fluorescence signals, magnetic nanoparticle labels configured to generate magnetic signals, or any other label configured to generate a detectable signal. Sandwich-type lateral flow assays include a labeled antibody deposited at a sample reservoir on a solid substrate. After sample is applied to the sample reservoir, the labeled antibody dissolves in the sample, whereupon the antibody recognizes and binds a first epitope on the analyte in the sample, forming an label-antibody-analyte complex. This complex flows along the liquid front from the sample reservoir through the solid substrate to a capture zone (sometimes referred to as a "test line"), where immobilized antibodies (sometimes referred to as "capture agent") are located. In some cases where the analyte is a multimer or contains multiple identical epitopes on the same monomer, the labeled antibody deposited at the sample reservoir can be the same as the antibody immobilized in the capture zone. The immobilized antibody recognizes and binds an epitope on the analyte, thereby capturing label-antibody-analyte complex at the capture zone. The presence of labeled antibody at the capture zone provides a detectable optical signal at the capture zone. In one non-limiting example, gold nanoparticles are used to label the antibodies because they are relatively inexpensive, stable, and provide easily observable color indications based on the surface plasmon resonance properties of gold nanoparticles. In some cases, this signal provides qualitative information, such as whether or not the analyte is present in the sample. In some cases, this signal provides quantitative information, such as a measurement of the quantity of analyte in the sample.

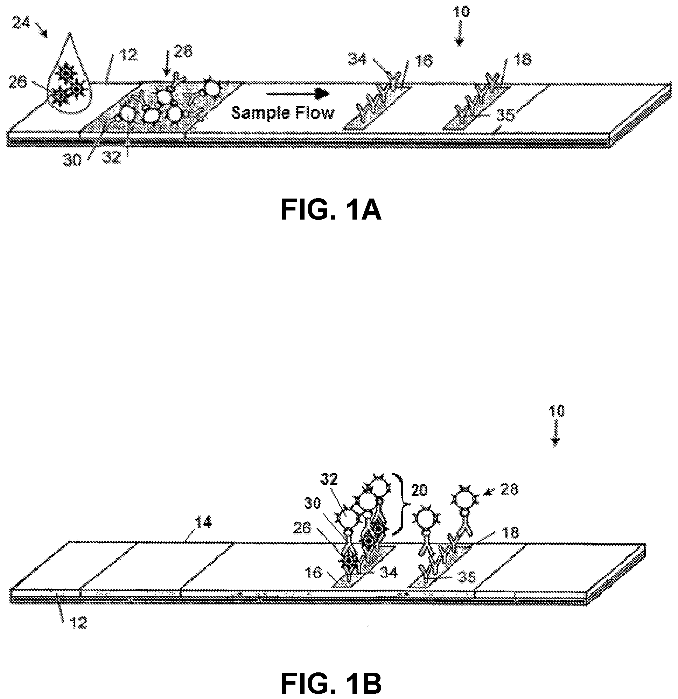

Figures 1A and 1B illustrate an example sandwich-type lateral flow device 10. The lateral flow device 10 includes a sample reservoir 12, a label zone 14, a capture zone 16, and a control line 18. Figures 1A and 1B illustrate the lateral flow device 10 before and after a fluid sample 24 has been applied to the sample reservoir 12. In the example illustrated in Figures 1A and 1B, the sample 24 includes analyte of interest 26. The label zone 14 that is in or near the sample reservoir 12 includes a labeled agent 28. In this example sandwich-type lateral flow device, the labeled agent 28 includes an antibody or antibody fragment 30 bound to a label 32. A capture agent 34 is immobilized in the capture zone 16. A control agent 35 is immobilized on the control line 18.

When the fluid sample 24 is applied to the sample reservoir 12, the sample 24 solubilizes the labeled agent 28, and the labeled agent 28 binds to analyte 26, forming an label-antibody-analyte complex 20. Accordingly, in the example sandwich-type lateral flow device 10, the label-antibody-analyte complex 20 is not formed until after the fluid sample 24 containing the analyte of interest 26 is applied to the lateral flow device. Further, in the example sandwich-type lateral flow device 10, the analyte in the label-antibody-analyte complex 20 is analyte from the fluid sample 24. As shown in Figure 1B, this complex 20 flows through the test strip to the capture zone 16, where it is bound by the capture agent 34. The now-bound complex 20 (and specifically, the label 32 on the now-bound complex 20) emits a detectable optical signal at the capture zone 16.

Labeled agent 28 that did not bind to any analyte 26 passes through the capture zone 16 (there being no analyte 26 to bind to a capture agent 34 in the capture zone 16) and continues to flow down the lateral flow device 10. In lateral flow assays that include the control line 18 such as that illustrated here, the deposited control agent 35 captures labeled agent 28 that did not bind to analyte 26 and passed through the capture zone 16 to the control line 18. In some embodiments, the control agent 35 captures the labeled agent 28 at the Fc region of the antibody. In some embodiments, the control agent 35 captures the labeled agent 28 at the Fab region of the antibody. This labeled agent 28 bound at the control line 18 emits a detectable optical signal that can be measured and used to indicate that the assay operated as intended (for example, the sample 24 flowed from the sample reservoir 12 and through the capture zone 16 as intended during normal operation of the lateral flow assay). One disadvantage of the example sandwich-type lateral flow device 10 is that the intensity of the signal generated at the control line 18 is dependent on the intensity of the signal generated at the capture zone 16 (because the control agent 35 at the control line 18 captures labeled agent 28 that did not bind to analyte 26 in the capture zone 16 and then passed to the control line 18). For example, if a relatively large amount of analyte 26 binds in the capture zone 16, a relatively small amount of analyte 26 will pass through the capture zone 16 and be available to bind to control agent 35 at the control line 18, resulting in a relatively weaker intensity signal at the control line 18.

Lateral flow assays can provide qualitative information, such as information on the absence or presence of the analyte of interest in the sample. For example, detection of any measurable optical signal at the capture zone 16 can indicate that the analyte of interest is present in the sample (in some unknown quantity). The absence of any measurable optical signal at the capture zone can indicate that the analyte of interest is not present in the sample or below the detection limit. For example, if the sample 24 did not contain any analyte of interest 26 (not illustrated), the sample 24 would still solubilize the labeled agent 28 and the labeled agent 28 would still flow to the capture zone 16. The labeled agent 28 would not bind to the capture agent 34 at the capture zone 16, however. It would instead flow through the capture zone 16, through the control line 18, and, in some cases, to an optional absorbing zone. Some labeled agent 28 would bind to the control agent 35 deposited on the control line 18 and emit a detectable optical signal. In these circumstances, the absence of a measureable optical signal emanating from the capture zone 16 is an indication that the analyte of interest is not present in the sample 24, and the presence of a measureable optical signal emanating from the control line 18 is an indication that the sample 24 traveled from the sample receiving zone 12, through the capture zone 16, and to the capture line 18 as intended during normal operation of the lateral flow assay.

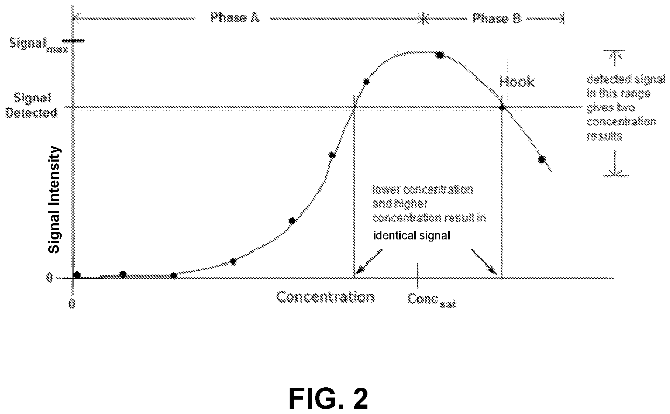

Some lateral flow devices can provide quantitative information, such as a measurement of the quantity of analyte of interest in the sample. The quantitative measurement obtained from the lateral flow device may be a concentration of the analyte that is present in a given volume of sample. Figure 2 illustrates an example quantitative measurement obtained from the sandwich-type lateral flow assay illustrated in Figures 1A and 1B. Figure 2 is a dose response curve that graphically illustrates the relationship between an intensity of a signal detected at the capture zone (measured along the y-axis) and the concentration of analyte in the sample (measured along the x-axis). Example signals include optical signals, fluorescence signals, and magnetic signals.

As shown by the first data point at zero concentration in Figure 2, if the sample does not contain any analyte of interest, the concentration of analyte in the sample is zero and no analyte binds to the labeled agent to form a label-antibody-analyte complex. In this situation, there are no complexes that flow to the capture zone and bind to the capture antibody. Thus, no detectable optical signal is observed at the capture zone and the signal magnitude is zero.

A signal is detected as the concentration of analyte in the sample increases from zero concentration. As demonstrated by data points in Phase A, the signal increases with increased analyte concentration in the sample. This takes place because as the analyte concentration increases, the formation of label-antibody-analyte complex increases. Capture agent immobilized at the capture zone binds the increasing number of complexes flowing to the capture zone, resulting in an increase in the signal detected at the capture zone. In Phase A, the signal continues to increase as the concentration of the analyte in the sample increases.

In some instances, if a sample has a concentration of analyte that exceeds the amount of labeled agent available to bind to the analyte, excess analyte is present. In these circumstances, excess analyte that is not bound by labeled agent competes with the label-antibody-analyte complex to bind to the capture agent in the capture zone. The capture agent in the capture zone will bind to un-labeled analyte (in other words, analyte not bound to a labeled agent) and to label-antibody-analyte complex. Un-labeled analyte that binds to the capture agent does not emit a detectable signal, however. As the concentration of analyte in the sample increases in Phase B, the amount of un-labeled analyte that binds to the capture agent (in lieu of a label-antibody-analyte complex that emits a detectable signal) increases. As more and more un-labeled analyte binds to the capture agent in lieu of label-antibody-analyte complex, the signal detected at the capture zone decreases, as shown by data points in Phase B.

This phenomenon where the detected signal increases during Phase A and the detected signal decreases in Phase B is referred to as a "hook effect." As the concentration of analyte increases in the Phase A, more analyte binds to the labeled agent, resulting in increased signal strength. At a point "Concₛₐₜ," the labeled agent is saturated with analyte from the sample (for example, the available quantity of labeled agent has all or nearly all bound to analyte from the sample), and the detected signal has reached a maximum value Signalₘₐₓ. As the concentration of the analyte in the sample continues to increase in Phase B, there is a decrease in the detected signal as excess analyte above the labeled agent saturation point competes with the labeled agent-analyte to bind to the capture agent.

The hook effect, also referred to as "the prozone effect," adversely affects lateral flow assays, particularly in situations where the analyte of interest is present in the sample at a concentration in Phase B. The hook effect can lead to inaccurate test results. For example, the hook effect can result in false negatives or inaccurately low results. Specifically, inaccurate results occur when a sample contains elevated levels of analyte that exceed the concentration of labeled agent deposited on the test strip. In this scenario, when the sample is placed on the test strip, the labeled agent becomes saturated, and not all of the analyte becomes labeled. The unlabeled analyte flows through the assay and binds at the capture zone, out-competing the labeled complex, and thereby reducing the detectable signal. Thus, the device (or the operator of the device) is unable to distinguish whether the optical signal corresponds to a low or a high concentration, as the single detected signal corresponds to both a low and a high concentration. If analyte levels are great enough, then the analyte completely out-competes the labeled complex, and no signal is observed at the capture zone, resulting in a false negative test result.

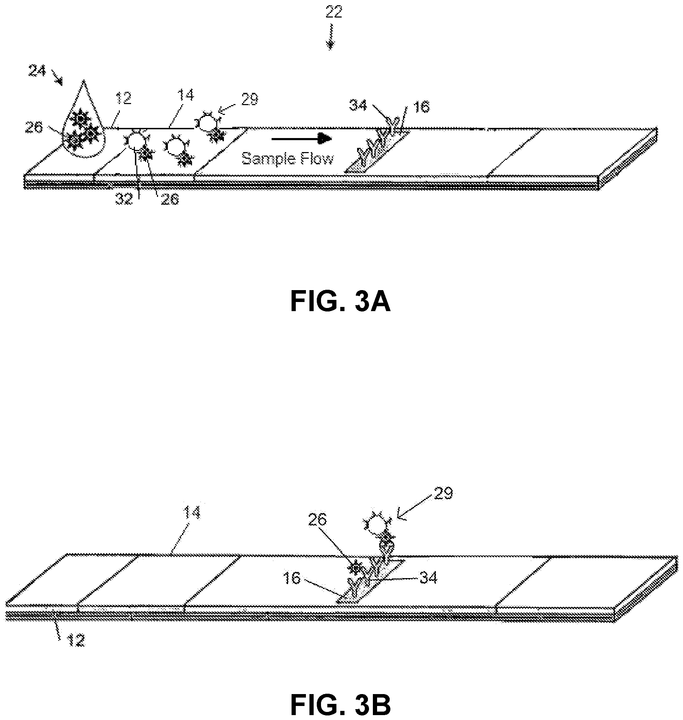

Inaccurate test results can also result from competitive-type lateral flow assays. In contrast to sandwich-type lateral flow assays, in a competitive-type lateral flow assay the un-labeled analyte of interest from a sample competes with labeled analyte of interest to bind to a capture agent at the capture zone. Figures 3A and 3B illustrate an example competitive-type lateral flow assay 22. The lateral flow device 22 includes a sample reservoir 12, a label zone 14, and a capture zone 16. Figures 3A and 3B illustrate the lateral flow device 22 before and after a fluid sample 24 has been applied to the sample reservoir 12. In the example illustrated in Figures 3A and 3B, the fluid sample 24 includes analyte of interest 26. The label zone 14 that is in or near the sample reservoir 12 includes a labeled agent 29. In this example competitive-type lateral flow device, the labeled agent 29 includes an analyte of interest 26 bound to a label 32. A capture agent 34 is immobilized in the capture zone 16.

The sample 24 that includes un-labeled analyte 26 is applied to the sample reservoir 12. The sample 24 solubilizes the labeled agent 29. The un-labeled analyte 26 in the sample 24 and the labeled agent 29 flow together to the capture zone 16, where both unlabeled analyte 26 from the sample 24 and labeled agent 29 bind to the capture agent 34 immobilized in the capture zone 16. As shown in Figure 3B, the labeled agent and the unlabeled analyte 26 compete with each other to bind to a fixed amount of capture agent 34. Labeled agent 29 bound to capture agent 34 (and specifically, the label 32 in labeled agent 29) emits a detectable optical signal, whereas un-labeled analyte 26 that originated from sample 24 and bound to capture agent 34 does not emit a detectable optical signal.

Detection of an optical signal from the capture zone 16 can provide qualitative or quantitative information about the analyte of interest 26. In the case where fluid sample 24 does not include any analyte 26 (not illustrated), the sample 24 would still solubilize the labeled agent 29 and the labeled agent 29 would still flow to the capture zone 16. The capture agent 34 in the capture zone 16 will bind to labeled agent 29 (which does not compete with any un-labeled analyte from the sample), resulting in a detected optical signal of maximum intensity or near maximum intensity. In a case where the sample 24 includes analyte 26 at very low or low concentration, an optical signal of maximum intensity or near maximum intensity may also be detected. This is because the proportion of un-labeled analyte 26 bound to capture agent 34 to labeled agent 29 bound to capture agent 34 will be low. Thus, it may be difficult to determine if a detected optical signal at maximum intensity should be correlate to zero concentration or low concentration of analyte 26 in the sample 24.

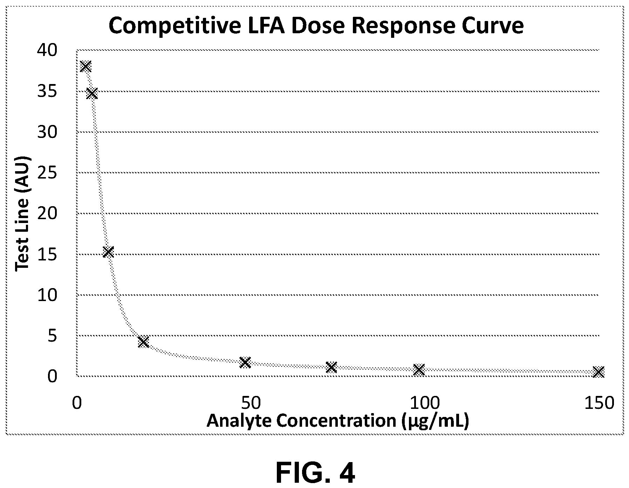

As the concentration of un-labeled analyte 26 increases in the sample 24, the detected optical signal emitted from the capture zone 16 decreases. This is because competition for the capture agent 34 increases with increasing analyte concentration in the sample, and the proportion of un-labeled analyte 26 bound to capture agent 34 to labeled agent 29 bound to capture agent 34 will progressively increase. If the analyte is present in the sample in high or very high concentrations, however, the optical signal detected at the capture zone 16 rapidly decreases to low magnitude signals. This rapid decrease in the strength of the optical signal as the concentration of analyte in the sample increases to high and very high concentrations makes it difficult if not impossible to precisely determine the concentration of the analyte, and in some cases renders the device inoperable to determine the concentration of the analyte at all. Competitive-type lateral flow devices such as that illustrated in Figures 3A and 3B are virtually incapable of accurately determining the precise concentration of the analyte of interest when the analyte of interest is present at high concentrations (for example, when the proportion of un-labeled analyte to labeled agent is high). Figure 4 illustrates a dose response curve generated in an example competitive-type lateral flow device such as that described above with reference to Figures 3A and 3B. As shown in Figure 4, the dose response curve of a competitive-type lateral flow assay exhibits a steep decrease in signal in concentrations of analyte ranging from about 1 to 20 µg/mL. Because of the steep decrease in the curve, the resolution is poor, decreasing the accuracy in determining quantities of analyte at high concentrations and, in some cases, making it impractical or virtually impossible to determine, with any degree of accuracy, a quantity of analyte present in a sample at high concentration.

### Example Lateral Flow Devices that Accurately Quantify an Analyte Present in a Sample at High Concentrations

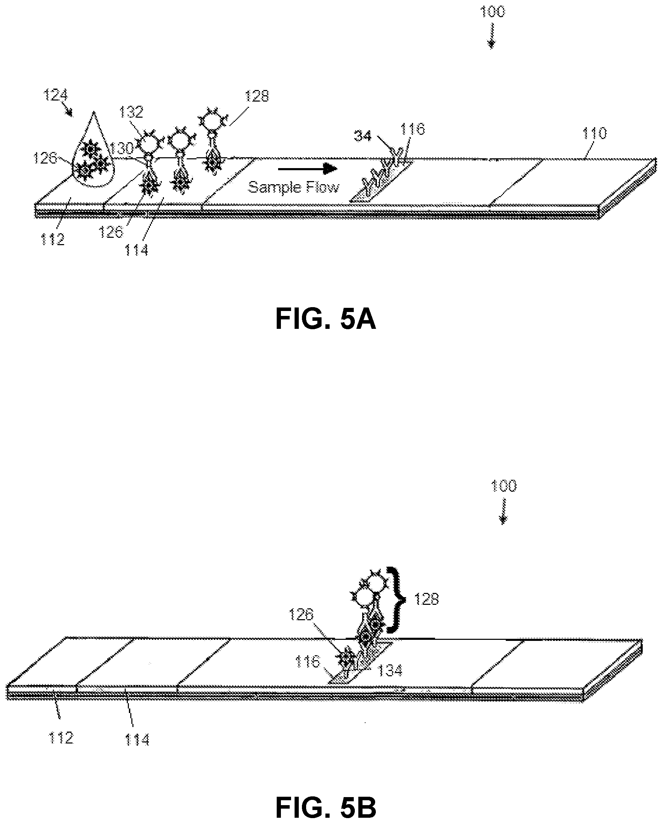

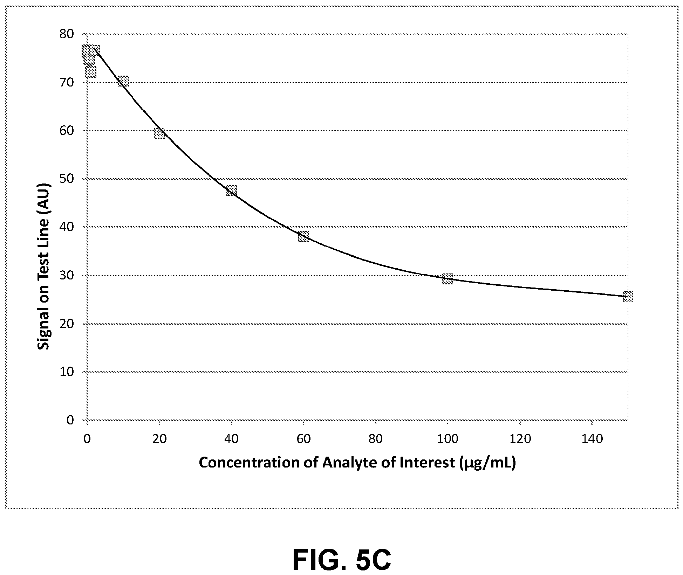

Lateral flow assays, test systems, and methods described herein address these and other drawbacks of sandwich-type and competitive-type lateral flow assays such as those illustrated in Figures 2A, 2B, 3A, and 3B. Figures 5A and 5B illustrate an example lateral flow assay 100 that can precisely measure a quantity of analyte of interest that is present in a sample at high concentrations. Figure 5C is an example dose response curve that graphically illustrates the optical signal measured from the lateral flow assay 100, and specifically the relationship between a magnitude of an optical signal detected at the capture zone (measured along the y-axis) and the concentration of analyte in the sample applied to the assay (measured along the x-axis). It will be understood that, although assays according to the present disclosure are described in the context of reflective-type labels generating optical signals, assays according to the present disclosure may include labels of any suitable material that are configured to generate fluorescence signals, magnetic signals, or any other detectable signal.

The lateral flow assay 100 includes a test strip 110 having a sample receiving zone 112, a label zone 114, and a capture zone 116. Figures 5A and 5B illustrate the lateral flow device 100 before and after a fluid sample 124 has been applied to a sample reservoir 112. In the illustrated example, the label zone 114 is downstream of the sample receiving zone 112 along a direction of sample flow 118 within the test strip 110. In some cases, the sample receiving zone 112 is located within and/or coextensive with the label zone 114. A capture agent 134 is immobilized in the capture zone 116.

A labeled agent 128 is integrated on the label zone 114. In lateral flow devices according to the present disclosure such as the non-limiting example discussed with reference to Figures 5A and 5B, the labeled agent 128 includes at least three components bound together to form a complex: a label (detector molecule) 132, an analyte of interest 126, and an antibody or fragment of an antibody 130 specific to the analyte of interest 126. The labeled agent 128 is a label-antibody-analyte complex 128. In some cases, the labeled agent 128 is formed and applied to the test strip 110 prior to use of the test strip 110 by an operator. For example, the labeled agent 128 can be integrated in the label zone 114 during manufacture of the test strip 110. In another example, the labeled agent 128 is integrated in the label zone 114 after manufacture but prior to application of the fluid sample to the test strip 110. The labeled agent 128 can be integrated into the test strip 110 in a number of ways discussed in greater detail below.

Accordingly, in embodiments of the lateral flow device of the present disclosure, a label-antibody-analyte complex 128 is formed and integrated on the test strip 110 before any fluid sample 124 has been applied to the lateral flow device. In one non-limiting example, the label-antibody-analyte complex 128 is formed and integrated onto the conjugate pad of the test strip 110 before any fluid sample 124 is applied to the lateral flow device. Further, in embodiments of the lateral flow device of the present disclosure, the analyte in the label-antibody-analyte complex 128 is not analyte from the fluid sample 124.

To perform a test using the test strip 110, a sample 124 that may or may not include analyte of interest 126 is deposited on the sample receiving zone 112. In the illustrated embodiment where the label zone 114 is downstream of the sample receiving zone 112, un-labeled analyte of interest 126 in the sample 124 next flows to the label zone 114 and comes into contact with the integrated labeled agent 128. The sample 124 solubilizes the labeled agent 128. In one non-limiting example, the sample 124 dissolves the labeled agent 128. The bonds that held the labeled agent 128 to the surface of the test strip 110 in the label zone 114 are released, so that the labeled agent 128 is no longer integrated onto the surface of the test strip 110. The labeled agent 128 next migrates with un-labeled analyte 126 in the sample 124 along the fluid front to the capture zone 116. Capture agent 134 at the capture zone 116 binds to labeled agent 128 and analyte 126 (if any) from the sample 124. Depending on the quantity of un-labeled analyte 126 in the sample 124, the labeled agent 128 and the un-labeled analyte 126 compete with each other to bind to capture agent 134 in the capture zone.

Accordingly, lateral flow devices according to the present disclosure have a labeled agent including an label-antibody-analyte complex that is bound to a label zone of the lateral flow device in a first phase (for example, prior to application of the fluid sample to the lateral flow device), and then migrates through the test strip in a second, later phase (for example, upon application of the fluid sample to the sample receiving zone). Labeled agents according to the present disclosure can bind to capture agents in the capture zone in a third phase (for example, after the fluid sample has flowed to the capture zone). Thus, labeled agents described herein can be initially positioned in a first region (such as a label zone) of a lateral flow device, then (upon contact with a fluid), migrate with the fluid to other regions of the lateral flow device downstream of the first region, and then bind to capture agents in the capture zone.

As described above, the fluid sample 124 solubilizes the labeled agent 128. In one implementation, the analyte of interest 126 in the sample 124 does not interact with, or does not interact substantially with, the labeled agent 128 during this process. Without being bound to any particular theory, in this implementation of the lateral flow devices described herein, the un-labeled analyte of interest 126 does not conjugate to, bind to, or associate with the labeled agent 128 as the sample 124 flows through the label zone 114. This is in contrast to the sandwich-type lateral flow device discussed above with reference to Figures 1A and 1B, where the labeled agent 28 binds to un-labeled analyte of interest 26 as the sample 24 flows through the label zone 14. In another implementation of the lateral flow devices described herein, the analyte of interest 126 in the sample 124 interacts with the labeled agent 128 when the fluid sample 124 solubilizes the labeled agent 128. Without being bound to any particular theory, in this implementation, capture agent 134 in the capture zone 116 may bind to at least some label-antibody-analyte complex where the analyte in the complex is analyte of interest 126 introduced onto the device via the sample 124.

When no analyte of interest 126 is present in the sample 124 (not illustrated), the labeled agent 128 saturates the capture agent 134 at the capture zone 116 (for example, every capture agent 134 molecule in the capture zone 135 binds to one labeled agent 128 that flowed from the label zone 114). The labeled agent 128 captured in the capture zone 116 emits a detectable optical signal that is the maximum intensity signal that can be obtained from the lateral flow device 100. The optical signal detected at the capture zone 116 in a scenario where no analyte of interest 126 is present in the sample 124 is referred to herein as being a "maximum intensity signal" because every available capture agent 134 at the capture zone 116 has bound to a labeled agent 128. In the non-limiting example illustrated in Figure 5C, the maximum intensity signal that is obtained when the concentration of analyte of interest is zero is at or about 76 AU (arbitrary signal intensity units).

There are many methods to determine the maximum intensity signal of the lateral flow device 100. In one non-limiting example, the maximum intensity signal that can be obtained from a particular lateral flow device 100 can be determined empirically and stored in a look-up table. In some cases, the maximum intensity signal is determined empirically by testing lateral flow devices 100 of known features and construction, for example by averaging the maximum intensity signal obtained when a sample having a zero or almost zero concentration of the analyte of interest is applied to lateral flow devices 100 of known specifications and construction. In another non-limiting example, the maximum intensity signal that can be obtained from a particular lateral flow device 100 can be determined using theoretical calculations given the known specifications and construction of the lateral flow device 100 (such as, for example, the amount and specific characteristics of the labeled agent 128 integrated on the label zone 114).

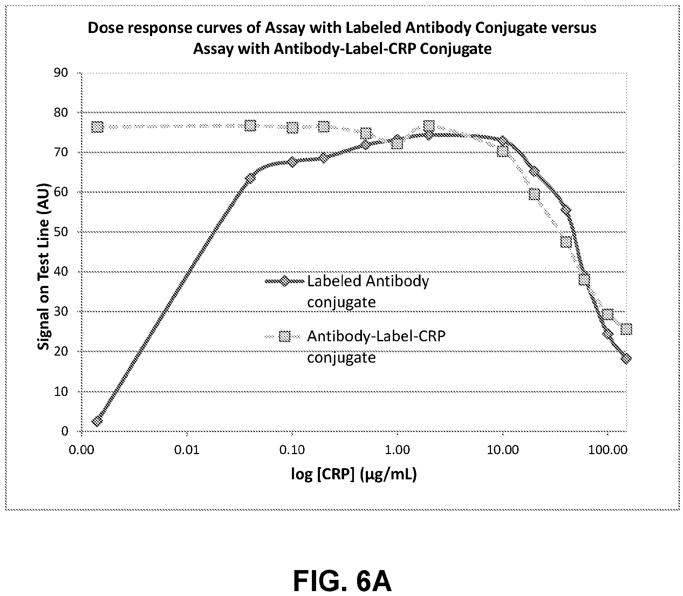

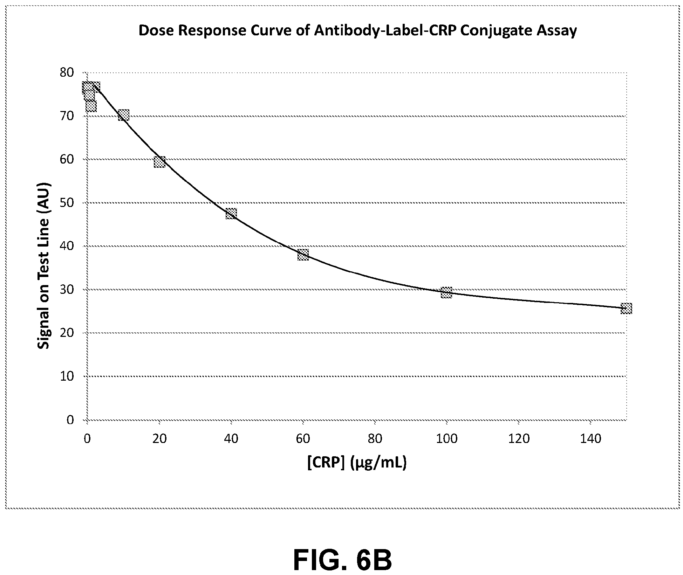

Further, it will be understood that although reference is made herein to "maximum intensity signal," signals that are within a particular range of the expected maximum intensity can be deemed substantially equivalent to the "maximum intensity signal." In addition, it will be understood that "maximum intensity signal" may refer to a maximum intensity optical signal, maximum intensity fluorescence signal, maximum intensity magnetic signal, or any other type of signal occurring at maximum intensity. As one non-limiting example, a detected signal that is within 1% of the expected maximum intensity signal is deemed substantially equivalent to the expected maximum intensity signal. If the maximum intensity signal is at or about 76 AU, a detected signal within a range of about 75.24 AU to about 76.76 AU would be deemed substantially equivalent to the maximum intensity signal of 76 AU. As another example, in the non-limiting embodiment described with reference to Figures 5C, 6A, and 6B, a detected signal that is within 10% of the expected maximum intensity signal is deemed substantially equivalent to the expected maximum intensity signal. Thus, in the example illustrated in Figure 5C where the maximum intensity signal is at or about 76 AU, a detected signal within the range of about 68.4 AU to about 83.6 AU is deemed substantially equivalent to the maximum intensity signal of 76 AU. These examples are provided for illustrative purposes only, as other variances may be acceptable. For instance, in lateral flow assay device according to the present disclosure, a detected signal that is within any suitable range of variance from the expected maximum intensity signal (such as but not limited to within 1.1%, 1.2%, 1.3%, 1.4%, 1.5%, 2.0%, 2.5%, 3%, 3.5%, 4%, 4.5%, 5%, 5.5%, 6%, 6.5%, 7%, 7.5%, 8%, 8.5%, 9%, 9.5%, 10%, 11%, 12%, 13%, 14%, 15% of the expected maximum intensity signal) can be deemed substantially equivalent to the expected maximum intensity signal.

In a scenario such as that illustrated in Figures 5A and 5B where analyte of interest 126 is present in the sample 124, the labeled agent 128 from the label zone 114 and the analyte 126 from the sample flow to the capture zone 116 where they compete to bind with capture agent 134. In one example, analyte of interest 126 is present in the sample 124 at a low concentration. An analyte of interest 126 can be deemed to be present in the sample 124 at low concentration when the detected optical signal at the capture zone 116 is the same, substantially the same, and/or within a particular range of variance from the maximum intensity signal. In one non-limiting example, the analyte of interest 126 is deemed to be present in the sample at low concentrations when the detected optical signal is within 5% of 76 AU (or within about 72.2 AU to about 79.8 AU). Optical signals within 5% of 76 AU correlate to concentrations of analyte of interest between 0 and about 1 µg/mL, such that concentrations between 0 and about 1 µg/mL would be considered low concentrations of the analyte of interest in this example. In the non-limiting example illustrated in Figure 5C, the analyte of interest 126 is deemed to be present in the sample at low concentrations when the detected optical signal is within 10% of 76 AU (or within about 68.4 AU to about 83.6 AU). Optical signals within 10% of 76 AU correlate to concentrations of analyte of interest between 0 and about 10 µg/mL, such that concentrations between 0 and about 10 µg/mL would be considered low concentrations of the analyte of interest in this example. In such low concentration cases where there is relatively little analyte of interest 126 in the sample 124, the proportion of analyte of interest 126 from the sample 124 that bound to capture agent relative to labeled agent 128 that bound to the capture agent is low. In such cases of low concentration, the optical signal detected at the capture zone 116 will be the same as or slightly less than the maximum intensity signal that would have been detected had there been no analyte of interest 126 in the sample 124.

As the concentration of analyte 126 in the sample 124 increases from about 1µg/mL to 10 µg/mL then to 20 µg/mL and greater concentrations, more analyte 126 is present at the capture zone 116 to compete with labeled agent 128 to bind to capture agent 134. This results in less labeled agent 128 binding at the capture zone 134 as the concentration of analyte 126 increases, and the detected optical signal at the capture zone 116 decreases.

As illustrated in Figure 5C, the decrease in the signal as the concentration of analyte of interest increases is advantageously gradual in embodiments of lateral flow devices according to the present disclosure. As a result of this gradual decrease in the detected signal, embodiments of lateral flow devices described herein advantageously allow a detector to precisely measure the signal with high resolution and a data analyzer to determine, with high precision, the concentration of the analyte of interest when the concentration is high. This is in contrast to competitive-type lateral flow devices described above with reference to Figures 3A, 3B, and 4.

In addition, the dose response curve of lateral flow devices according to the present disclosure advantageously begin at a maximum intensity signal and then decrease from this maximum intensity signal. This means that, advantageously, no signal in the portion of the dose response curve where the signal is decreasing will have a magnitude that is the same as the maximum intensity signal. Further, because the signal when the concentration of analyte in the sample is low will be the same as or effectively the same as the maximum intensity signal (for example, they are deemed substantially equivalent to the maximum intensity signals as described above), there is a plateau of optical signals at a relatively constant value ("maximum intensity signal") for zero to low concentrations of analyte (as will be discussed in detail below with reference to non-limiting examples). This means that, advantageously, no signal in the portion of the dose response curve where the signal is decreasing will have a magnitude that is about the same as the maximum intensity signal. False negatives and inaccurately low readings are thus avoided in embodiments of the lateral flow devices described herein. This is in contrast to the sandwich-type lateral flow device discussed above with reference to Figure 1A, 1B, and 2, where a high concentration of analyte in the sample will generate a signal that is the same as or about the same as a signal generated when the concentration of analyte is low.

Advantageously, in embodiments of lateral flow devices described herein, the labeled agent 128 can be pre-formulated to include a known quantity of analyte of interest prior to deposition on the conjugate pad. In some embodiments, analyte of interest of a known concentration is incubated with an antibody or fragment of an antibody and label molecules in a reaction vessel that is separate from the test strip. During incubation, the analyte of interest becomes conjugated to, bound to, or associated with the antibody and label molecules to form a labeled agent 128 as described above. After incubation, the labeled agent 128 is either directly added to a solution at a precise, known concentration or isolated to remove excess free CRP before being sprayed onto the conjugate pad. The solution including the labeled agent 128 is applied to the test strip, such as on the label zone 114 described above. During deposition, the labeled agent 128 becomes integrated on the surface of the test strip. In one non-limiting example, the labeled agent is integrated onto the conjugate pad of the test strip. Advantageously, labeled agent 128 can remain physically bound to and chemically stable on the surface of the test strip until an operator applies a fluid sample to the test strip, whereupon the labeled agent 128 unbinds from the test strip and flows with the fluid sample as described above.

In some embodiments, the labeled agent 128 is deposited in an amount ranging from about 0.1-20 µL/test strip. In some embodiments, the labeled agent 128 is deposited in an amount of 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10, 11, 12, 13, 14, 15, 16, 17, 18, 19, or 20 µL/test strip in the label zone.

The solution including the labeled agent 128 can be applied to the test strip in many different ways. In one example, the solution is applied to the label zone 114 by spraying the solution with airjet techniques. In another example, the solution including the labeled agent 128 is deposited by pouring the solution, spraying the solution, formulating the solution as a power or gel that is placed or rubbed on the test strip, or any other suitable method to apply the isolated labeled agent 128. In some embodiments, after deposition, the labeled agent 128 is dried on the surface of the test strip after deposition by heating or blowing air on the conjugate pad. Other mechanisms to dry the labeled agent 128 on the surface of the test strip are suitable. For example, vacuum or lypholization can also be used to dry the labeled agent 128 on the conjugate pad. In some cases, the isolated labeled agent 128 is not added to a solution prior to deposition and is instead applied directly to the test strip. The labeled agent 128 can be directly applied using any suitable method, including but not limited to applying compressive or vacuum pressure to the labeled agent 128 on the surface of the test strip and/or applying labeled agent 128 in the form of lyophilized particles to the surface of the test strip.

Embodiments of the lateral flow assay illustrated in Figures 5A and 5B need not include a control line or zone configured to confirm that a sample applied in the sample receiving zone 112 has flowed to the capture zone 116 as intended. Under normal operating circumstances, some detectable signal will always be emitted from the capture zone 116 if the sample has flowed to the capture zone 116. This will be the case even if the analyte of interest is present in the sample at extremely low concentrations, because the lateral flow devices of the present disclosure have a dose response curve that remains at or near a maximum intensity signal for low concentrations. Therefore, the absence of any detectable signal at the capture zone 116 after the sample has been applied to the sample receiving zone 112 can be used an indication that the lateral flow assay did not operate as intended (for example, the sample did not flow to the capture zone 116 as intended, or as another example, the immobilized capture agents 134 at the capture zone are defective or faulty). Accordingly, a further advantage of embodiments of lateral flow devices according to the present disclosure is the ability of the capture zone to function as a control line, thereby permitting a separate control line to be omitted from the test strip altogether. It will be understood, however, that a control line could be included in embodiments of lateral flow devices described herein for a variety of purposes, including but not limited to a viewing line, for normalizing noise, or for detecting interference from analytes in serum.

In some cases, lateral flow assays according to the present disclosure include a control line, such as a control line similar to control line 18 described above with reference to Figures 1A and 1B. In one embodiment (not illustrated), the lateral flow assay includes a control line including a capture reagent that emits a signal whose intensity is independent of the intensity of the intensity of the signal generated by the labeled agent 128 in the capture zone 116. In one implementation, the lateral flow assay includes a plurality of capture zones (including at least one capture zone 116 configured to capture a labeled agent 128 according to the present disclosure), each capture zone configured to indicate the presence, absence, and/or concentration of a different analyte of interest, and a single control line configured to indicate that the sample flowed through the plurality of capture zones as intended. In contrast to the control line 18 described above with reference to Figures 1A and 1B, the intensity of the signal emanating from the control line in this implementation may not be related to or dependent on the intensity of the signal emanating from any of the capture zones. Embodiments that include a control line may also be advantageous in instances where the capture zone 116 emits a signal of relatively weak intensity when the analyte of interest is present in the sample at extremely high concentration. In such cases, the signal emanating from the capture zone 116 may be of insufficient intensity to confirm that the assay operated as intended (for example that the sample flowed through the capture zone 116 as intended).

Further, multiplex assays that test for the presence, absence, and/or quantity of a plurality of different analytes of interest can include a lateral flow assay according to the present disclosure (as described above with reference to Figures 5A and 5B) on the same test strip as one or more sandwich-type lateral flow assays as described above with reference to Figure 1A and 1B. In such multiplex assays, even though a control line is not needed for the lateral flow assay according to the present disclosure, a control line may still be advantageously included on the test strip to confirm that the sample has flowed through the control zone associated with a sandwich-type lateral flow assay. This option to include a control line for one assay and to omit a control line for an assay according to the present disclosure can be particularly beneficial in multiplex assays where there are a limited number of lines or zones that can be positioned on the test strip.

The following non-limiting examples illustrate features of lateral flow devices, test systems, and methods described herein, and are in no way intended to limit the scope of the present disclosure.

### Example 1

### Preparation of a Lateral Flow Assay to Quantify Elevated Protein Concentration

The following example describes preparation of a lateral flow assay to quantify an analyte of interest as described herein. In this non-limiting example, the analyte of interest is a protein, C-reactive protein (CRP), present in a serum sample at an elevated or high concentration.

CRP is a protein found in blood plasma. Levels of CRP rise in response to inflammation. CRP is thus a marker for inflammation that can be used to screen for inflammation. Elevated levels of CRP in the serum of a subject can be correlated to inflammation, viral infection, and/or bacterial infection in the subject. Normal levels of CRP in healthy human subjects range from about 1 µg/mL to about 10 µg/mL. Concentrations of CRP during mild inflammation and viral infection range from 10-40 µg/mL; during active inflammation and bacterial infection from 40-200 µg/mL; and in severe bacterial infections and burn cases greater than 200 µg/mL. Measuring and charting CRP levels be useful in determining disease progress or the effectiveness of treatments.

The assay prepared according to this non-limiting example can be used to determine the precise concentration of CRP (the analyte of interest) in a serum sample even when the concentration is above normal levels of CRP in healthy human subjects (about 1 µg/mL to about 10 µg/mL). The assay includes a labeled agent including an antibody-label-CRP complex that avoids several drawbacks of sandwich-type lateral flow assays, including drawbacks associated with the hook effect.

To prepare the assay, anti-C-reactive protein (anti-CRP) antibody was incubated with gold nanoparticles to form labeled anti-CRP antibody. The labeled antibody was incubated with CRP to form a complex of labeled antibody bound to CRP. The complex was deposited in an amount of 1.8 µL/test strip onto a conjugate pad (label zone) by spraying a solution including the complex with airjet. The conjugate pad was heated to dry the complex to the conjugate pad.

The amount of antibody-label-CRP complex deposited on the conjugate pad was carefully considered to ensure a requisite amount of complex to provide an optimal range of optical signals at the capture zone that will allow a test system to quantify elevated levels of CRP. Depositing an excess amount of complex on the conjugate pad will shift the dose response curve, such that the quantifiable concentration of CRP is excessively high (potentially generating optical signals for very high concentrations of CRP (if present) but not generating optical signals for mild to high concentrations). Depositing an insufficient amount of complex on the conjugate pad shifts the dose response curve in the other direction, resulting in signals that may not allow quantification of very high CRP concentrations. Table 1 demonstrates the results of experiments to determine an optimal amount of antibody-label-CRP complex to deposit on the conjugate pad. The amount of pre-formed label-antibody-analyte complex deposited on the conjugate pad can vary to accommodate the requirement of different concentration ranges of analytes.

**Table 1: Optical Signal Intensity for Various Amounts of Antibody-Label-CRP Complex on the Conjugate Pad**

| Amount of CRP (ng) per test added to the conjugate pad | CRP Line Intensity (AU) |
|---|---|
| 0 | 0.36 |
| 5 | 74.02 |
| 7.5 | 77.93 |
| 10 | 75.10 |
| 15 | 75.76 |
| 20 | 76.69 |
| 30 | 75.37 |
| 50 | 70.06 |
| 100 | 67.17 |
| 200 | 44.17 |

In this example, the optimal amount of antibody-label-CRP complex to add to the conjugate pad results in 50 ng of CRP deposited on the conjugate pad, corresponding to a signal of 70.06 AU. At this amount, the ratio of unlabeled CRP in the sample to antibody-label-CRP complex as they compete to bind to the capture agent in the capture zone generates a strong optical signal over an optimal range of unlabeled CRP concentrations, thereby allowing for adequate resolution of the signal, and elevated CRP concentration in a sample can be accurately quantified. Advantageously, depositing 50 ng of CRP at the conjugate pad (through deposition of an appropriate amount of antibody-label-CRP complex at the conjugate pad) results in ratio of unlabeled CRP to labeled agent (antibody-label-CRP complex) that would be on the portion of a sandwich-type assay dose response curve having decreasing optical signals (for example, in Phase B of Figure 2). This ratio of unlabeled CRP to labeled agent (antibody-label-CRP complex) also allows the lateral flow assay in this example to mask the portion of a sandwich-type assay dose response curve having increasing optical signals (for example, in Phase A of Figure 2). Without being bound to any particular theory, it is believed that embodiments of the lateral flow assay in this example effectively mask the increasing optical signal portion of a sandwich-type lateral flow assay by adding an optimized amount of CRP (in this example, 50 ng) to the conjugate pad, using only portions of the dose response curve exhibiting decreasing signal intensity (the portion of the curve exhibiting the "hook effect") and thereby avoiding disadvantages described above with reference to Figures 1A, 1B, and 2.

In this example, anti-CRP antibody was deposited at the capture zone in an amount of 2 mg/mL. Goat anti-mouse antibody was deposited at a control zone in an amount of 2 mg/mL.

### Example 2

### Quantification of High Concentration C-Reactive Protein using a Lateral Flow Assay

Due to the hook effect, sandwich-type lateral flow assays such as those described above with reference to Figures 1A and 1B are generally unsuitable to quantify the concentration of CRP when it is present at elevated levels in a sample. To determine elevated concentrations previously required serial dilutions of the sample, resulting in an inefficient and laborious process. Using lateral flow devices, test systems, and methods described herein, however, concentrations of CRP above healthy levels can be accurately, reliably, and quickly quantified.

Lateral flow assays as prepared in Example 1 were contacted with a sample including various concentrations of CRP, as shown in the last column of Table 2 below. In this example, the amount of antibody-label-CRP complex added to the conjugate pad resulted in 100 ng of CRP deposited on the conjugate pad. Sandwich-type lateral flow assays such as those described above with reference to Figures 1A and 1B were contacted with identical samples, as shown in the middle column of Table 2. As described above, the sandwich-type lateral flow assays referenced in the middle column only included a labeled antibody deposited on the conjugate pad (no CRP deposited on the conjugate pad via an antibody-label-CRP complex). Fluid samples were prepared by adding the amounts of CRP shown in the first column of Table 2 in 30 µL of human serum. The sample was received on the lateral flow assay, and after 15 seconds, chased with 45 µL of HEPES buffer. After ten minutes, the optical signal was measured. All samples were run in sextuplicate, and the average values are reported in Table 2. Figure 6A illustrates the resulting dose response curves for the lateral flow assay with labeled antibody deposited on the conjugate pad (solid line with diamonds) and the lateral flow assay with antibody-label-CRP complex deposited on the conjugate pad (dashed line with squares), where concentration of analyte is measured along the x-axis in logarithmic scale. Figure 6B illustrates the example dose response curve of Figure 6A for the lateral flow assay with antibody-label-CRP complex, where the concentration of analyte is measured along the x-axis in non-logarithmic scale.

**Table 2: Comparison of Traditional Sandwich-Type Lateral Flow Assay and Lateral Flow Assay of the Present Disclosure**

| Amount of Unlabeled CRP (µg/mL) in Serum Sample | Signal (AU) of Lateral Flow Assay with Labeled Antibody Complex Added to Conjugate Pad | Signal (AU) of Lateral Flow Assay with Antibody-Label-CRP Complex Added to Conjugate Pad (100 ng CRP per test) |
|---|---|---|
| 0.0014 | 2.49 | 76.37 |
| 0.04 | 63.48 | 76.70 |
| 0.10 | 67.56 | 76.20 |
| 0.20 | 68.60 | 76.51 |
| 0.50 | 71.89 | 74.83 |
| 1 | 73.19 | 72.20 |
| 2 | 74.38 | 76.61 |
| 10 | 72.96 | 70.29 |
| 20 | 65.24 | 59.43 |
| 40 | 55.57 | 47.51 |
| 60 | 39.05 | 38.00 |
| 100 | 24.39 | 29.33 |
| 150 | 18.21 | 25.61 |

**Figure 6A** highlights significant differences between a sandwich-type lateral flow assay that includes the hook effect and lateral flow assays according to the present disclosure. In the sandwich-type lateral flow assay that includes the hook effect, concentrations of CRP greater than 10 µg/mL (1.00 in logarithmic scale) generate optical signals that are the same intensity as concentrations of CRP less than 10 µg/mL. In contrast, the lateral flow assay according to the present disclosure allows the concentration of CRP to be accurately determined at concentrations greater than 10 µg/mL. This is particularly advantageous in the present example where the analyte of interest is CRP, which elevates to concentrations greater than 10 µg/mL when inflammation or disease conditions are present. Embodiments of the lateral flow assays described herein allow a user to determine with confidence that the concentration of CRP in the subject under test is above normal levels. When a test according to the present disclosure is performed and indicates a concentration of CRP greater than healthy levels (for example, greater than 10 µg/mL), this information can be correlated to an inflammation, viral infection, and/or bacterial infection condition.

Further, the ability to accurately pinpoint the precise concentration of CRP in the subject under test can allow the test result to be correlated to a specific type of disease condition. For example, a concentration between 10 µg/mL and 20 µg/mL may be correlated to mild inflammation whereas a concentration between 40 µg/mL and 200 µg/mL may be correlated to a bacterial infection. In addition, the ability to accurately pinpoint the precise concentration of CRP in the subject under test may allow the test result to be correlated to a stage of disease. For example, a concentration between 40 µg/mL and 200 µg/mL may be correlated to mild bacterial infection whereas a concentration greater than 200 µg/mL may be correlated to a severe bacterial infection. These examples are illustrative and are not intended to limit the scope of the present disclosure.

Lateral flow assay devices, systems, and methods disclosed herein provide additional advantages. For example, the lateral flow assay according to the present disclosure is capable of reliable quantification of an analyte in a sample by using portions of the dose response curve that exhibit the hook effect. As illustrated in Figure 6A, concentrations of CRP at or below healthy levels (about 10 µg/mL or less) result in a signal that is at or within 10% of a maximum intensity of 76 AU (76.20 AU to 70.29 AU). Thus, samples of low concentration generate a plateau of signals at relatively constant values (in this case, signals within 10% of the maximum intensity signal of 76 AU). In embodiments of the lateral flow assay according to the present disclosure, this overlap in optical signals for concentrations of CRP at low concentrations is not a drawback because low concentrations of CRP are always present in healthy subjects and the test need not be sensitive to CRP concentrations at low levels.

Instead, the lateral flow assay of the present disclosure is, advantageously, particularly sensitive to analyte of interest present at high concentrations. High concentrations of analyte generate signals that are not on or near the plateau, in this case signals that are less than about 70 AU. The lateral flow assay generates gradually decreasing signals when the concentration of CRP is above healthy levels (greater than 10 µg/mL), where the signals are readily detectable (discernable signal strength and well spaced apart) and do not overlap with other signals on the dose response curve. This eliminates the uncertainty of determining a quantity of analyte at a particular detected signal, such as in sandwich-type lateral flow assays that generate a signal value that can correspond to more than one quantity of analyte due to the hook effect. In such circumstances, the user is unable to determine whether the concentration of analyte is low or high, resulting in uncertainty for purposes of diagnosis. In contrast, the lateral flow assay according to the present disclosure generates a signal that clearly and unambiguously corresponds to a zero or low concentration of analyte (signal at or substantially equivalent to the maximum intensity signal) or a high concentration of analyte (signal less than the maximum intensity signal), which can then be directly correlated to a normal level of analyte (zero or low concentration of analyte) or a non-normal level of analyte (high concentration of analyte).

Furthermore, lateral flow devices described herein quantify elevated concentrations of an analyte in a sample in one single assay, without the need to dilute the sample. Assays such as those described with reference to Figures 1A, 1B, 3A, and 3B, in contrast, require dilution of samples that include high concentrations of analyte; otherwise, the signals of the high-concentration portion of the dose response curve are indistinguishable. The lateral flow assay of the present disclosure is capable of determining even minute differences in elevated analyte concentration based on a single signal obtained at the capture zone after one test.

### Example 3

### CRP Concentration Measured Using Lateral Flow Assays According to the Present Disclosure are Highly Correlated to ELISA Assays

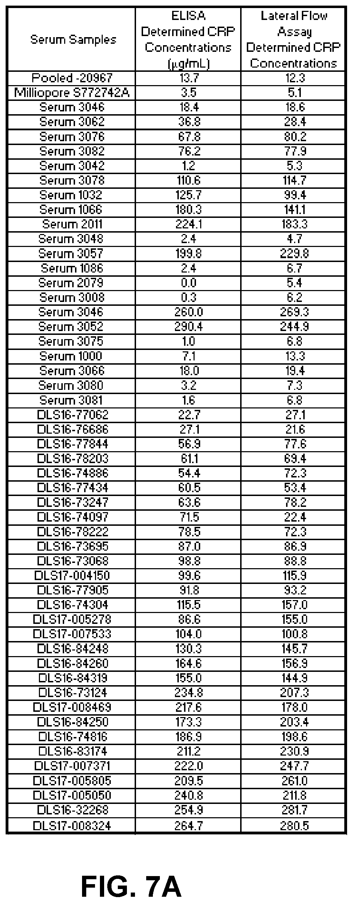

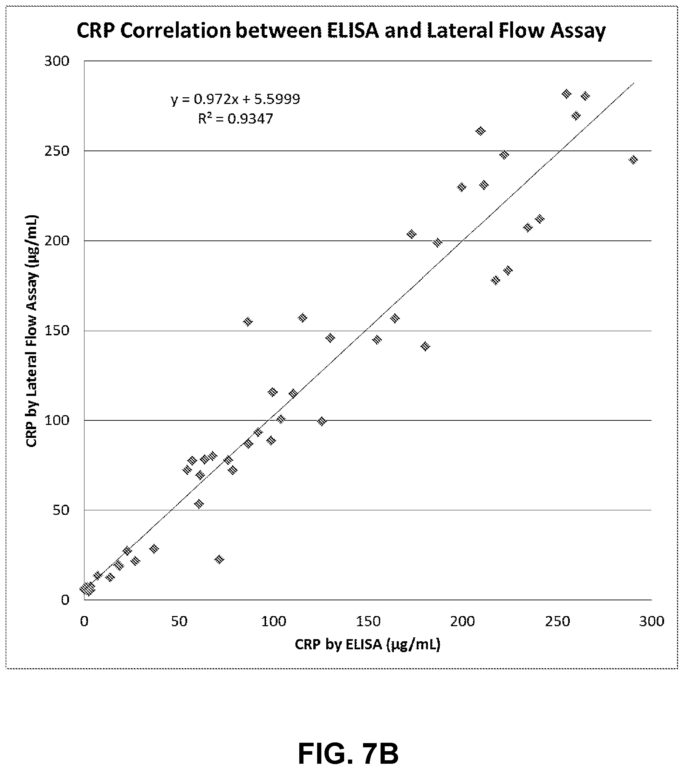

Furthermore, embodiments of the lateral flow assay according to the present disclosure strongly correlate with current gold standard assays for determining the quantity of analyte in a sample, such as enzyme-linked immunosorbent assay (ELISA). Advantageously, the concentration of CRP as determined by embodiments of the lateral flow assays described herein has been discovered to strongly correlate with the concentration of CRP as determined by ELISA. Figure 7A is a table summarizing the concentration of CRP in various serum samples measured using lateral flow assays according to the present disclosure and the concentration of CRP in the same serum samples measured using ELISA. Figure 7B is a chart correlating the CRP concentrations obtained in accordance with the present disclosure and CRP concentrations measured by ELISA. As illustrated in Figure 7B, a correlation of 93% between concentration of CRP measured using embodiments of assays according to the present disclosure and concentration of CRP determined by ELISA was obtained.

### Methods of Diagnosing a Condition Using Lateral Flow Assays According to the Present Disclosure

Some embodiments provided herein relate to methods of using lateral flow assays to diagnose a medical condition. In some embodiments, the method includes providing a lateral flow assay as described herein. In some embodiments, the method includes receiving a sample at a sample reservoir of the lateral flow assay.

In some embodiments, the sample is obtained from a source, including an environmental or biological source. In some embodiments, the sample is suspected of having an analyte of interest. In some embodiments, the sample is not suspected of having an analyte of interest. In some embodiments, a sample is obtained and analyzed for verification of the absence or presence of an analyte. In some embodiments, a sample is obtained and analyzed for the quantity of analyte in the sample. In some embodiments, the quantity of an analyte in a sample is less than a normal value present in healthy subjects, at or around a normal value present in healthy subjects, or above a normal value present in healthy subjects.

In some embodiments, receiving a sample at the sample reservoir of the lateral flow assay includes contacting a sample with a lateral flow assay. A sample may contact a lateral flow assay by introducing a sample to a sample reservoir by external application, as with a dropper or other applicator. In some embodiments, a sample reservoir may be directly immersed in the sample, such as when a test strip is dipped into a container holding a sample. In some embodiments, a sample may be poured, dripped, sprayed, placed, or otherwise contacted with the sample reservoir.

A labeled agent in embodiments of the present disclosure include an antibody, a label, and an analyte of interest and can be deposited on a conjugate pad (or label zone) within or downstream of the sample reservoir. The labeled agent can be integrated on the conjugate pad by physical or chemical bonds. The sample solubilizes the labeled agent after the sample is added to the sample reservoir, releasing the bonds holding the labeled agent to the conjugate pad. The sample, including analyte (if present) and the labeled agent flow along the fluid front through the lateral flow assay to a capture zone. Capture agent immobilized at the capture zone binds analyte (if present) and the labeled agent. When labeled agent binds to capture agent at the capture zone, a signal from the label is detected. The signal may include an optical signal as described herein. When low concentrations of analyte are present in the sample (such as levels at or below healthy levels), a maximum intensity signal at the capture zone is detected. At elevated concentrations of analyte (such as levels above healthy values), the intensity of the detected signal decreases in an amount proportionate to the amount of analyte in the sample. The detected signal is compared to values on a dose response curve for the analyte of interest, and the concentration of analyte in the sample is determined.

In some embodiments, the analyte is present in elevated concentrations. Elevated concentrations of analyte can refer to a concentration of analyte that is above healthy levels. Thus, elevated concentration of analyte can include a concentration of analyte that is 5%, 10%, 15%, 20%, 25%, 30%, 35%, 40%, 50%, 60%, 70%, 80%, 90%, 100%, 125%, 150%, 200%, or greater than a healthy level. In some embodiments, an analyte of interest includes C-reactive protein (CRP), which is present in blood serum of healthy individuals in an amount of about 1 to about 10 µg/mL. Thus, elevated concentrations of CRP in a sample includes an amount of 15, 20, 25, 30, 35, 40, 45, 50, 55, 60, 65, 70, 75, 80, 85, 90, 95, 100, 110, 120, 130, 140, 150, 160, 170, 180, 190, or 200 µg/mL or greater. The level at which an analyte of interest will be considered elevated may differ depending on the specific analyte of interest.

In some embodiments, upon determination that an analyte is present in a sample in elevated concentrations, the subject is diagnosed with a certain disease. In some embodiments, diagnosis of an infection is made when the concentration of CRP is deteremined to be 15, 20, 25, 30, 35, 40, 45, 50, 55, 60, 65, 70, 75, 80, 85, 90, 95, 100, 110, 120, 130, 140, 150, 160, 170, 180, 190, or 200 µg/mL or greater. In some embodiments, a determination that the concentration is greater than 200 µg/mL, for example, 400-500 µg/mL, results in a diagnosis of severe bacterial infection.

### Example Test Systems Including Lateral Flow Assays According to the Present Disclosure

Lateral flow assay test systems described herein can include a lateral flow assay test device (such as but not limited to a test strip), a housing including a port configured to receive all or a portion of the test device, a reader including a light source and a light detector, a data analyzer, and combinations thereof. A housing may be made of any one of a wide variety of materials, including plastic, metal, or composite materials. The housing forms a protective enclosure for components of the diagnostic test system. The housing also defines a receptacle that mechanically registers the test strip with respect to the reader. The receptacle may be designed to receive any one of a wide variety of different types of test strips. In some embodiments, the housing is a portable device that allows for the ability to perform a lateral flow assay in a variety of environments, including on the bench, in the field, in the home, or in a facility for domestic, commercial, or environmental applications.

A reader may include one or more optoelectronic components for optically inspecting the exposed areas of the capture zone of the test strip. In some implementations, the reader includes at least one light source and at least one light detector. In some embodiments, the light source may include a semiconductor light-emitting diode and the light detector may include a semiconductor photodiode. Depending on the nature of the label that is used by the test strip, the light source may be designed to emit light within a particular wavelength range or light with a particular polarization. For example, if the label is a fluorescent label, such as a quantum dot, the light source would be designed to illuminate the exposed areas of the capture zone of the test strip with light in a wavelength range that induces fluorescent emission from the label. Similarly, the light detector may be designed to selectively capture light from the exposed areas of the capture zone. For example, if the label is a fluorescent label, the light detector would be designed to selectively capture light within the wavelength range of the fluorescent light emitted by the label or with light of a particular polarization. On the other hand, if the label is a reflective-type label, the light detector would be designed to selectively capture light within the wavelength range of the light emitted by the light source. To these ends, the light detector may include one or more optical filters that define the wavelength ranges or polarizations axes of the captured light. A signal from a label can be analyzed, using visual observation or a spectrophotometer to detect color from a chromogenic substrate; a radiation counter to detect radiation, such as a gamma counter for detection of ¹²⁵I; or a fluorometer to detect fluorescence in the presence of light of a certain wavelength. Where an enzyme-linked assay is used, quantitative analysis of the amount of an analyte of interest can be performed using a spectrophotometer. Lateral flow assays described herein can be automated or performed robotically, if desired, and the signal from multiple samples can be detected simultaneously. Furthermore, multiple signals can be detected in a multiplex-type assay, where more than one analyte of interest is detected, identified, or quantified.

A multiplex assay could include, for example, a viral differential assay. For example, a multiplex lateral flow assay as described herein can detect whether one or several viral proteins are present in a sample from a subject suffering from a viral infection or suspected of suffering from a viral infection (for example, exhibiting flu-like symptoms). In some embodiments, a multiplex lateral flow assay for this purpose would be capable of detecting elevated concentrations of CRP and low concentrations of TRAIL and IP-10.

The data analyzer processes the signal measurements that are obtained by the reader. In general, the data analyzer may be implemented in any computing or processing environment, including in digital electronic circuitry or in computer hardware, firmware, or software. In some embodiments, the data analyzer includes a processor (e.g., a microcontroller, a microprocessor, or ASIC) and an analog-to-digital converter. The data analyzer can be incorporated within the housing of the diagnostic test system. In other embodiments, the data analyzer is located in a separate device, such as a computer, that may communicate with the diagnostic test system over a wired or wireless connection. The data analyzer may also include circuits for transfer of results via a wireless connection to an external source for data analysis or for reviewing the results.

In general, the results indicator may include any one of a wide variety of different mechanisms for indicating one or more results of an assay test. In some implementations, the results indicator includes one or more lights (e.g., light-emitting diodes) that are activated to indicate, for example, the completion of the assay test. In other implementations, the results indicator includes an alphanumeric display (e.g., a two or three character light-emitting diode array) for presenting assay test results.

Test systems described herein can include a power supply that supplies power to the active components of the diagnostic test system, including the reader, the data analyzer, and the results indicator. The power supply may be implemented by, for example, a replaceable battery or a rechargeable battery. In other embodiments, the diagnostic test system may be powered by an external host device (e.g., a computer connected by a USB cable).

### Features of Example Lateral Flow Devices

Lateral flow devices described herein can include a sample reservoir (also referred to as a sample receiving zone) where a fluid sample is introduced to a test strip, such as but not limited to an immunochromatographic test strip present in a lateral flow device. In one example, the sample may be introduced to sample reservoir by external application, as with a dropper or other applicator. The sample may be poured or expressed onto the sample reservoir. In another example, the sample reservoir may be directly immersed in the sample, such as when a test strip is dipped into a container holding a sample.

Lateral flow devices described herein can include a solid support or substrate. Suitable solid supports include but are not limited to nitrocellulose, the walls of wells of a reaction tray, multi-well plates, test tubes, polystyrene beads, magnetic beads, membranes, and microparticles (such as latex particles). Any suitable porous material with sufficient porosity to allow access by labeled agents and a suitable surface affinity to immobilize capture agents can be used in lateral flow devices described herein. For example, the porous structure of nitrocellulose has excellent absorption and adsorption qualities for a wide variety of reagents, for instance, capture agents. Nylon possesses similar characteristics and is also suitable. Microporous structures are useful, as are materials with gel structure in the hydrated state.

Further examples of useful solid supports include: natural polymeric carbohydrates and their synthetically modified, cross-linked or substituted derivatives, such as agar, agarose, cross-linked alginic acid, substituted and cross-linked guar gums, cellulose esters, especially with nitric acid and carboxylic acids, mixed cellulose esters, and cellulose ethers; natural polymers containing nitrogen, such as proteins and derivatives, including cross-linked or modified gelatins; natural hydrocarbon polymers, such as latex and rubber; synthetic polymers which may be prepared with suitably porous structures, such as vinyl polymers, including polyethylene, polypropylene, polystyrene, polyvinylchloride, polyvinylacetate and its partially hydrolyzed derivatives, polyacrylamides, polymethacrylates, copolymers and terpolymers of the above polycondensates, such as polyesters, polyamides, and other polymers, such as polyurethanes or polyepoxides; porous inorganic materials such as sulfates or carbonates of alkaline earth metals and magnesium, including barium sulfate, calcium sulfate, calcium carbonate, silicates of alkali and alkaline earth metals, aluminum and magnesium; and aluminum or silicon oxides or hydrates, such as clays, alumina, talc, kaolin, zeolite, silica gel, or glass (these materials may be used as filters with the above polymeric materials); and mixtures or copolymers of the above classes, such as graft copolymers obtained by initializing polymerization of synthetic polymers on a pre-existing natural polymer.

Lateral flow devices described herein can include porous solid supports, such as nitrocellulose, in the form of sheets or strips. The thickness of such sheets or strips may vary within wide limits, for example, from about 0.01 to 0.5 mm, from about 0.02 to 0.45 mm, from about 0.05 to 0.3 mm, from about 0.075 to 0.25 mm, from about 0.1 to 0.2 mm, or from about 0.11 to 0.15 mm. The pore size of such sheets or strips may similarly vary within wide limits, for example from about 0.025 to 15 microns, or more specifically from about 0.1 to 3 microns; however, pore size is not intended to be a limiting factor in selection of the solid support. The flow rate of a solid support, where applicable, can also vary within wide limits, for example from about 12.5 to 90 sec/cm (i.e., 50 to 300 sec/4 cm), about 22.5 to 62.5 sec/cm (i.e., 90 to 250 sec/4 cm), about 25 to 62.5 sec/cm (i.e., 100 to 250 sec/4 cm), about 37.5 to 62.5 sec/cm (i.e., 150 to 250 sec/4 cm), or about 50 to 62.5 sec/cm (i.e., 200 to 250 sec/4 cm). In specific embodiments of devices described herein, the flow rate is about 35 sec/cm (i.e., 140 sec/4 cm). In other specific embodiments of devices described herein, the flow rate is about 37.5 sec/cm (i.e., 150 sec/4 cm).

The surface of a solid support may be activated by chemical processes that cause covalent linkage of an agent (e.g., a capture reagent) to the support. As described below, the solid support can include a conjugate pad. Many other suitable methods may be used for immobilizing an agent (e.g., a capture reagent) to a solid support including, without limitation, ionic interactions, hydrophobic interactions, covalent interactions and the like.

Except as otherwise physically constrained, a solid support may be used in any suitable shapes, such as films, sheets, strips, or plates, or it may be coated onto or bonded or laminated to appropriate inert carriers, such as paper, glass, plastic films, or fabrics.

Lateral flow devices described herein can include a conjugate pad, such as a membrane or other type of material that includes a capture reagent. The conjugate pad can be a cellulose acetate, cellulose nitrate, polyamide, polycarbonate, glass fiber, membrane, polyethersulfone, regenerated cellulose (RC), polytetra-fluorethylene, (PTFE), Polyester (e.g. Polyethylene Terephthalate), Polycarbonate (e.g., 4,4-hydroxy-diphenyl-2,2'-propane), Aluminum Oxide, Mixed Cellulose Ester (e.g., mixture of cellulose acetate and cellulose nitrate), Nylon (e.g., Polyamide, Hexamethylene-diamine, and Nylon 66), Polypropylene, PVDF, High Density Polyethylene (HDPE)+nucleating agent "aluminum dibenzoate" (DBS) (e.g. 80 u 0.024 HDPE DBS (Porex)), and HDPE.

Lateral flow devices described herein are highly sensitive to an analyte of interest that is present in a sample at high concentrations. As described above, high concentrations are present when unlabeled analyte of interest in the sample is present in an amount sufficient to compete with a labeled compound to bind to a capture agent in the capture zone, resulting in a detected signal on a negative-slope portion of a dose response curve (for example, on the "hook effect" portion of the dose response curve of a conventional sandwich-type lateral flow assay or a negative-slope portion of a dose response curve according to lateral flow assays of the present disclosure). "Sensitivity" refers to the proportion of actual positives which are correctly identified as such (for example, the percentage of infected, latent or symptomatic subjects who are correctly identified as having a condition). Sensitivity may be calculated as the number of true positives divided by the sum of the number of true positives and the number of false negatives.

Lateral flow devices described herein can accurately measure an analyte of interest in many different kinds of samples. Samples can include a specimen or culture obtained from any source, as well as biological and environmental samples. Biological samples may be obtained from animals (including humans) and encompass fluids, solids, tissues, and gases. Biological samples include urine, saliva, and blood products, such as plasma, serum and the like. Such examples are not however to be construed as limiting the sample types applicable to the present disclosure.

In some embodiments the sample is an environmental sample for detecting an analyte in the environment. In some embodiments, the sample is a biological sample from a subject. In some embodiments, a biological sample can include peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen (including prostatic fluid), Cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, or other lavage fluids.

As used herein, "analyte" generally refers to a substance to be detected. For instance, analytes may include antigenic substances, haptens, antibodies, and combinations thereof. Analytes include, but are not limited to, toxins, organic compounds, proteins, peptides, microorganisms, amino acids, nucleic acids, hormones, steroids, vitamins, drugs (including those administered for therapeutic purposes as well as those administered for illicit purposes), drug intermediaries or byproducts, bacteria, virus particles, and metabolites of or antibodies to any of the above substances. Specific examples of some analytes include ferritin; creatinine kinase MB (CK-MB); human chorionic gonadotropin (hCG); digoxin; phenytoin; phenobarbital; carbamazepine; vancomycin; gentamycin; theophylline; valproic acid; quinidine; luteinizing hormone (LH); follicle stimulating hormone (FSH); estradiol, progesterone; C-reactive protein (CRP); lipocalins; IgE antibodies; cytokines; TNF-related apoptosis-inducing ligand (TRAIL); vitamin B2 micro-globulin; interferon gamma-induced protein 10 (IP-10); glycated hemoglobin (Gly Hb); cortisol; digitoxin; N-acetylprocainamide (NAPA); procainamide; antibodies to rubella, such as rubella-IgG and rubella IgM; antibodies to toxoplasmosis, such as toxoplasmosis IgG (Toxo-IgG) and toxoplasmosis IgM (Toxo-IgM); testosterone; salicylates; acetaminophen; hepatitis B virus surface antigen (HBsAg); antibodies to hepatitis B core antigen, such as anti-hepatitis B core antigen IgG and IgM (Anti-HBC); human immune deficiency virus 1 and 2 (HIV 1 and 2); human T-cell leukemia virus 1 and 2 (HTLV); hepatitis B e antigen (HBeAg); antibodies to hepatitis B e antigen (Anti-HBe); influenza virus; thyroid stimulating hormone (TSH); thyroxine (T4); total triiodothyronine (Total T3); free triiodothyronine (Free T3); carcinoembryoic antigen (CEA); lipoproteins, cholesterol, and triglycerides; and alpha fetoprotein (AFP). Drugs of abuse and controlled substances include, but are not intended to be limited to, amphetamine; methamphetamine; barbiturates, such as amobarbital, secobarbital, pentobarbital, phenobarbital, and barbital; benzodiazepines, such as librium and valium; cannabinoids, such as hashish and marijuana; cocaine; fentanyl; LSD; methaqualone; opiates, such as heroin, morphine, codeine, hydromorphone, hydrocodone, methadone, oxycodone, oxymorphone and opium; phencyclidine; and propoxyhene. Additional analytes may be included for purposes of biological or environmental substances of interest.

Lateral flow devices described herein can include a label. Labels can take many different forms, including a molecule or composition bound or capable of being bound to an analyte, analyte analog, detector reagent, or binding partner that is detectable by spectroscopic, photochemical, biochemical, immunochemical, electrical, optical or chemical means. Examples of labels include enzymes, colloidal gold particles (also referred to as gold nanoparticles), colored latex particles, radioactive isotopes, co-factors, ligands, chemiluminescent or fluorescent agents, protein-adsorbed silver particles, protein-adsorbed iron particles, protein-adsorbed copper particles, protein-adsorbed selenium particles, protein-adsorbed sulfur particles, protein-adsorbed tellurium particles, protein-adsorbed carbon particles, and protein-coupled dye sacs. The attachment of a compound (e.g., a detector reagent) to a label can be through covalent bonds, adsorption processes, hydrophobic and/or electrostatic bonds, as in chelates and the like, or combinations of these bonds and interactions and/or may involve a linking group.

The term "specific binding partner (or binding partner)" refers to a member of a pair of molecules that interacts by means of specific, noncovalent interactions that depend on the three-dimensional structures of the molecules involved. Typical pairs of specific binding partners include antigen/antibody, hapten/antibody, hormone/receptor, nucleic acid strand/complementary nucleic acid strand, substrate/enzyme, inhibitor/enzyme, carbohydrate/lectin, biotin/(strept)avidin, receptor/ligands, and virus/cellular receptor, or various combinations thereof.

As used herein, the terms "immunoglobulin" or "antibody" refer to proteins that bind a specific antigen. Immunoglobulins include, but are not limited to, polyclonal, monoclonal, chimeric, and humanized antibodies, Fab fragments, F(ab')2 fragments, and includes immunoglobulins of the following classes: IgG, IgA, IgM, IgD, IbE, and secreted immunoglobulins (sIg). Immunoglobulins generally comprise two identical heavy chains and two light chains. However, the terms "antibody" and "immunoglobulin" also encompass single chain antibodies and two chain antibodies.

Lateral flow devices described herein include a labeled agent. In some cases, a labeled agent includes a detection agent that is capable of binding to an analyte. The labeled agent can be specific for an analyte. In some embodiments, a labeled agent can be an antibody or fragment thereof that has been conjugated to, bound to, or associated with a detection agent. In embodiments of the lateral flow assays according to the present disclosure, a labeled agent can be an antibody or fragment thereof that has been conjugated to, bound to, or associated with a detection agent and an analyte of interest, forming an label-antibody-analyte complex.

Lateral flow devices according to the present disclosure include a capture agent. A capture agent includes an immobilized agent that is capable of binding to an analyte, including a free (unlabeled) analyte and/or a labeled analyte. A capture agent includes an unlabeled specific binding partner that is specific for (i) a labeled analyte of interest, (ii) a labeled analyte or an unlabeled analyte, as in a competitive assay, or for (iii) an ancillary specific binding partner, which itself is specific for the analyte, as in an indirect assay. As used herein, an "ancillary specific binding partner" is a specific binding partner that binds to the specific binding partner of an analyte. For example, an ancillary specific binding partner may include an antibody specific for another antibody, for example, goat anti-human antibody. Lateral flow devices described herein can include a "capture area" that is a region of the lateral flow device where the capture reagent is immobilized. Lateral flow devices described herein may include more than one capture area, for example, a "primary capture area," a "secondary capture area," and so on. In some cases, a different capture reagent will be immobilized in the primary, secondary, and/or other capture areas. Multiple capture areas may have any orientation with respect to each other on the lateral flow substrate; for example, a primary capture area may be distal or proximal to a secondary (or other) capture area along the path of fluid flow and vice versa. Alternatively, a primary capture area and a secondary (or other) capture area may be aligned along an axis perpendicular to the path of fluid flow such that fluid contacts the capture areas at the same time or about the same time.

Lateral flow devices according to the present disclosure include capture agents that are immobilized such that movement of the capture agent is restricted during normal operation of the lateral flow device. For example, movement of an immobilized capture agent is restricted before and after a fluid sample is applied to the lateral flow device. Immobilization of capture agents can be accomplished by physical means such as barriers, electrostatic interactions, hydrogen-bonding, bioaffinity, covalent interactions or combinations thereof.

Lateral flow devices according to the present disclosure can include multiplex assays. Multiplex assays include assays in which multiple, different analytes of interest can be detected, identified, and in some cases quantified. For example, in a multiplex assay device, a primary, secondary, or more capture areas may be present, each specific for one analyte of interest of a plurality of analytes of interest.

Lateral flow devices according to the present disclosure can detect, identify, and in some cases quantify a biologic. A biologic includes chemical or biochemical compounds produced by a living organism which can include a prokaryotic cell line, a eukaryotic cell line, a mammalian cell line, a microbial cell line, an insect cell line, a plant cell line, a mixed cell line, a naturally occurring cell line, or a synthetically engineered cell line. A biologic can include large macromolecules such as proteins, polysaccharides, lipids, and nucleic acids, as well as small molecules such as primary metabolites, secondary metabolites, and natural products.

## Claims

1. A method of measuring an analyte of interest present in a fluid sample, the method comprising:
applying the fluid sample to an assay test strip, wherein the assay test strip comprises:
a flow path configured to receive a fluid sample;
a sample receiving zone coupled to the flow path;
a capture zone coupled to the flow path downstream of the sample receiving zone and comprising an immobilized capture agent specific to an analyte of interest; and
a complex coupled to the flow path and configured to flow in the flow path to the capture zone in the presence of the fluid sample, the complex comprising a label, an antibody or a fragment of an antibody that specifically binds the analyte of interest, and the analyte of interest;
uncoupling the complex from the flow path;
flowing the fluid sample and the complex in the flow path to the capture zone;
binding the complex to the immobilized capture agent in the capture zone; and
detecting a signal from the complex bound to the immobilized capture agent in the capture zone, wherein the detected signal is correlated to a measurement of a concentration of analyte of interest in the fluid sample.

2. The method of claim 1, wherein the fluid sample is a serum sample, and wherein the analyte of interest comprises C-reactive protein (CRP).

3. The method of claim 2, wherein the measured concentration of CRP present in the serum sample is in an amount ranging from about 10 µg/mL to greater than 200 µg/ml.

4. The method of any one of the preceding claims, wherein the detected signal is an optical signal, a fluorescence signal, or a magnetic signal.

5. The method of any one of the preceding claims, wherein uncoupling the complex comprises solubilizing the complex with the fluid sample.

6. The method of any one of the preceding claims, wherein the fluid sample comprises unlabeled analyte of interest, and wherein the complex does not specifically bind to the unlabeled analyte of interest.

7. The method of any one of the preceding claims, further comprising displaying an indication that the analyte of interest is present in the fluid sample.

8. The method of any one of the preceding claims, wherein detecting comprises detecting a signal from the test strip that is less than a maximum signal of a dose response curve of the test strip.

9. A method of manufacturing an assay test strip comprising:
coupling a sample receiving zone to a flow path configured to receive a fluid sample;
coupling a capture zone to the flow path downstream of the sample receiving zone; and
coupling a complex to the flow path, the complex comprising:
a label;
an antibody or a fragment of an antibody that specifically binds an analyte of interest; and
the analyte of interest.

10. The method of claim 9, wherein the fluid sample is a serum sample, and wherein the analyte of interest comprises C-reactive protein (CRP), and the antibody comprises anti-CRP antibody or a fragment of anti-CRP antibody.

11. The method of claim 9 or 10, wherein the analyte of interest comprises CRP in an amount of about 50 ng or about 100 ng.

12. The method of any one of claims 9 to 11, further comprising immobilizing a capture agent specific to the analyte of interest on the capture zone.

13. The method of any one of claims 9 to 12, wherein coupling the complex to the flow path comprises forming a bond between the complex and the flow path that breaks in the presence of fluid sample in the flow path.

14. The method of any one of claims 9 to 13, wherein coupling the complex comprises spraying a solution comprising the complex onto a surface of the sample receiving zone, optionally, wherein the complex is sprayed onto the surface of the assay test strip between the sample receiving zone and the capture zone.

15. The method of any one of claims 9 to 14, wherein coupling the complex comprises:
applying a fluid solution comprising the complex onto a surface of the assay test strip; and
drying the fluid solution.

16. The method of any one of claims 9 to 15, wherein coupling the complex comprises integrating the complex into a surface of the assay test strip.

17. The method of any one of claims 10 to 16, further comprising providing a solution comprising the complex.

18. The method of claim 17, wherein providing the solution comprises mixing a first liquid comprising the label and the antibody or fragment of the antibody with a second liquid comprising the analyte of interest.

19. The method of claim 18, wherein providing the solution further comprises incubating the mixture of the first liquid and the second liquid for about 30 minutes.

20. The method of any one of claims 9 to 19, wherein coupling the complex to the flow path comprises spraying the solution onto a surface of the assay test strip.

## Patentansprüche

1. Verfahren zur Messung eines Analyten von Interesse, der in einer Fluidprobe vorhanden ist, wobei das Verfahren umfasst:
Aufbringen der Fluidprobe auf einen Teststreifen, wobei der Teststreifen umfasst:
einen Strömungsweg zur Aufnahme einer Fluidprobe;
einen Probenaufnahmebereich, der mit dem Strömungsweg gekoppelt ist;
einen Erfassungsbereich, der mit dem Strömungsweg stromabwärts des Probenaufnahmebereichs gekoppelt ist und ein immobilisiertes Erfassungsmittel enthält, das für einen Analyten von Interesse spezifisch ist; und
einen Komplex, der mit dem Strömungsweg gekoppelt und dazu ausgelegt ist, in Gegenwart der Fluidprobe durch den Strömungsweg zum Erfassungsbereich zu strömen, wobei der Komplex einen Marker, einen Antikörper oder ein Fragment eines Antikörpers, der/das spezifisch den Analyten von Interesse bindet, und den Analyten von Interesse enthält;
Abkoppeln des Komplexes von dem Strömungsweg;
Strömen der Fluidprobe und des Komplexes in dem Strömungsweg zu dem Erfassungsbereich;
Binden des Komplexes an das immobilisierte Erfassungsmittel in dem Erfassungsbereich; und
Detektieren eines Signals von dem Komplex, der an das immobilisierte Erfassungsmittel in dem Erfassungsbereich gebunden ist, wobei das detektierte Signal mit einer Messung einer Konzentration des Analyten von Interesse in der Fluidprobe korreliert.

2. Verfahren nach Anspruch 1, wobei es sich bei der Fluidprobe um eine Serumprobe handelt und wobei der Analyt von Interesse C-reaktives Protein (CRP) enthält.

3. Verfahren nach Anspruch 2, wobei die gemessene Konzentration von CRP in der Fluidprobe in einer Menge von etwa 10 µg/ml bis über 200 µg/ml vorliegt.

4. Verfahren nach einem der vorhergehenden Ansprüche, wobei das detektierte Signal ein optisches Signal, ein Fluoreszenzsignal oder ein magnetisches Signal ist.

5. Verfahren nach einem der vorhergehenden Ansprüche, wobei das Abkoppeln des Komplexes die Solubilisierung des Komplexes mit der Fluidprobe umfasst.

6. Verfahren nach einem der vorhergehenden Ansprüche, wobei die Fluidprobe einen unmarkierten Analyten von Interesse enthält und wobei der Komplex nicht spezifisch an den unmarkierten Analyten von Interesse bindet.

7. Verfahren nach einem der vorhergehenden Ansprüche, ferner umfassend das Anzeigen einer Angabe, dass der Analyt von Interesse in der Fluidprobe vorhanden ist.

8. Verfahren nach einem der vorhergehenden Ansprüche, wobei das Detektieren das Detektieren eines Signals von dem Teststreifen umfasst, das geringer als ein maximales Signal einer Dosis-Wirkungs-Kurve des Teststreifens ist.

9. Verfahren zur Herstellung eines Teststreifens, umfassend:
Koppeln eines Probenaufnahmebereichs mit einem Strömungsweg zur Aufnahme einer Fluidprobe;
Koppeln eines Erfassungsbereichs mit dem Strömungsweg stromabwärts des Probenaufnahmebereichs; und
Koppeln eines Komplexes mit dem Strömungsweg, wobei der Komplex enthält:
einen Marker;
einen Antikörper oder ein Fragment eines Antikörpers, der/das spezifisch einen Analyten von Interesse bindet; und
den Analyten von Interesse.

10. Verfahren nach Anspruch 9, wobei es sich bei der Fluidprobe um eine Serumprobe handelt und wobei der Analyt von Interesse C-reaktives Protein (CRP) enthält und der Antikörper Anti-CRP-Antikörper oder ein Fragment eines Anti-CRP-Antikörper enthält.

11. Verfahren nach Anspruch 9 oder 10, wobei der Analyt von Interesse CRP in einer Menge von etwa 50 ng oder etwa 100 ng enthält.

12. Verfahren nach einem der Ansprüche 9 bis 11, ferner umfassend das Immobilisieren eines für den Analyten von Interesse spezifischen Erfassungsmittels in dem Erfassungsbereich.

13. Verfahren nach einem der Ansprüche 9 bis 12, wobei das Koppeln des Komplexes mit dem Strömungsweg das Bilden einer Bindung zwischen dem Komplex und dem Strömungsweg umfasst, die in Gegenwart einer Fluidprobe im Strömungsweg bricht.

14. Verfahren nach einem der Ansprüche 9 bis 13, wobei das Koppeln des Komplexes das Aufsprühen einer den Komplex enthaltenden Lösung auf eine Oberfläche des Probenaufnahmebereichs umfasst, wobei der Komplex optional auf die Oberfläche des Teststreifens zwischen dem Probenaufnahmebereich und dem Erfassungsbereich aufgesprüht wird.

15. Verfahren nach einem der Ansprüche 9 bis 14, wobei das Koppeln des Komplexes umfasst:
Aufbringen einer den Komplex enthaltenden Fluidlösung auf eine Oberfläche des Teststreifens; und
Trocknen der Fluidlösung.

16. Verfahren nach einem der Ansprüche 9 bis 15, wobei das Koppeln des Komplexes das Integrieren des Komplexes in eine Oberfläche des Teststreifens umfasst.

17. Verfahren nach einem der Ansprüche 10 bis 16, ferner umfassend das Bereitstellen einer den Komplex enthaltenden Lösung.

18. Verfahren nach Anspruch 17, wobei das Bereitstellen der Lösung das Mischen einer ersten Flüssigkeit, die den Marker und den Antikörper oder das Fragment des Antikörpers enthält, mit einer zweiten Flüssigkeit, die den Analyten von Interesse enthält, umfasst.

19. Verfahren nach Anspruch 18, wobei das Bereitstellen der Lösung ferner das Inkubieren der Mischung aus der ersten Flüssigkeit und der zweiten Flüssigkeit für etwa 30 Minuten umfasst.

20. Verfahren nach einem der Ansprüche 9 bis 19, wobei das Koppeln des Komplexes mit dem Strömungsweg das Aufsprühen der Lösung auf eine Oberfläche des Teststreifens umfasst.

## Revendications

1. Procédé de mesure d'un analyte d'intérêt présent dans un échantillon de fluide, le procédé comprenant :
l'application de l'échantillon de fluide sur une bandelette réactive de dosage, dans lequel la bandelette réactive de dosage comprend :
un trajet d'écoulement configuré pour recevoir un échantillon de fluide ;
une zone de réception d'échantillon couplée au trajet d'écoulement ;
une zone de capture couplée au trajet d'écoulement en aval de la zone de réception d'échantillon et comprenant un agent de capture immobilisé spécifique à un analyte d'intérêt ; et
un complexe couplé au trajet d'écoulement et configuré pour s'écouler sur le trajet d'écoulement vers la zone de capture en présence de l'échantillon de fluide, le complexe comprenant un marqueur, un anticorps ou un fragment d'un anticorps qui se lie spécifiquement à l'analyte d'intérêt, et l'analyte d'intérêt ;
le découplage du complexe du trajet d'écoulement ;
l'écoulement de l'échantillon de fluide et du complexe sur le trajet d'écoulement vers la zone de capture ;
la liaison du complexe à l'agent de capture immobilisé dans la zone de capture ; et
la détection d'un signal provenant du complexe lié à l'agent de capture immobilisé dans la zone de capture, dans lequel le signal détecté est corrélé à une mesure d'une concentration d'un analyte d'intérêt dans l'échantillon de fluide.

2. Procédé selon la revendication 1, dans lequel l'échantillon de fluide est un échantillon de sérum, et dans lequel l'analyte d'intérêt comprend une CRP [C-reactive protéine - protéine C réactive].

3. Procédé selon la revendication 2, dans lequel la concentration mesurée de CRP présente dans l'échantillon de sérum est en une quantité allant d'environ 10 µg/ml à plus de 200 µg/ml.

4. Procédé selon l'une quelconque des revendications précédentes, dans lequel le signal détecté est un signal optique, un signal de fluorescence ou un signal magnétique.

5. Procédé selon l'une quelconque des revendications précédentes, dans lequel le découplage du complexe comprend la solubilisation du complexe avec l'échantillon de fluide.

6. Procédé selon l'une quelconque des revendications précédentes, dans lequel l'échantillon de fluide comprend un analyte non marqué d'intérêt, et dans lequel le complexe ne se lie pas spécifiquement à l'analyte non marqué d'intérêt.

7. Procédé selon l'une quelconque des revendications précédentes, comprenant en outre l'affichage d'une indication que l'analyte d'intérêt est présent dans l'échantillon de fluide.

8. Procédé selon l'une quelconque des revendications précédentes, dans lequel la détection comprend la détection d'un signal provenant de la bandelette réactive qui est inférieur à un signal maximum d'une courbe dose-réponse de la bandelette réactive.

9. Procédé de fabrication d'une bandelette réactive de dosage comprenant :
le couplage d'une zone de réception d'échantillon à un trajet d'écoulement configuré pour recevoir un échantillon de fluide ;
le couplage d'une zone de capture au trajet d'écoulement en aval de la zone de réception d'échantillon ; et
le couplage d'un complexe au trajet d'écoulement, le complexe comprenant :
un marqueur ;
un anticorps ou un fragment d'un anticorps qui se lie spécifiquement à un analyte d'intérêt ; et
l'analyte d'intérêt.

10. Procédé selon la revendication 9, dans lequel l'échantillon de fluide est un échantillon de sérum, et dans lequel l'analyte d'intérêt comprend une CRP, et l'anticorps comprend un anticorps anti-CRP ou un fragment d'anticorps anti-CRP.

11. Procédé selon la revendication 9 ou 10, dans lequel l'analyte d'intérêt comprend la CRP en une quantité d'environ 50 ng ou d'environ 100 ng.

12. Procédé selon l'une quelconque des revendications 9 à 11, comprenant en outre l'immobilisation d'un agent de capture spécifique à l'analyte d'intérêt sur la zone de capture.

13. Procédé selon l'une quelconque des revendications 9 à 12, dans lequel le couplage du complexe au trajet d'écoulement comprend la formation d'une liaison entre le complexe et le trajet d'écoulement qui se rompt en présence de l'échantillon de fluide sur le trajet d'écoulement.

14. Procédé selon l'une quelconque des revendications 9 à 13, dans lequel le couplage du complexe comprend la pulvérisation d'une solution comprenant le complexe sur une surface de la zone de réception d'échantillon, dans lequel le complexe est optionnellement pulvérisé sur la surface de la bandelette réactive de dosage entre la zone de réception d'échantillon et la zone de capture.

15. Procédé selon l'une quelconque des revendications 9 à 14, dans lequel le couplage du complexe comprend :
l'application d'une solution fluide comprenant le complexe sur une surface de la bandelette réactive de dosage ; et
le séchage de la solution fluide.

16. Procédé selon l'une quelconque des revendications 9 à 15, dans lequel le couplage du complexe comprend l'intégration du complexe dans une surface de la bandelette réactive de dosage.

17. Procédé selon l'une quelconque des revendications 10 à 16, comprenant en outre la fourniture d'une solution comprenant le complexe.

18. Procédé selon la revendication 17, dans lequel la fourniture de la solution comprend le mélange d'un premier liquide comprenant le marqueur et l'anticorps ou le fragment de l'anticorps à un deuxième liquide comprenant l'analyte d'intérêt.

19. Procédé selon la revendication 18, dans lequel la fourniture de la solution comprend en outre l'incubation du mélange du premier liquide et du deuxième liquide pendant environ 30 minutes.

20. Procédé selon l'une quelconque des revendications 9 à 19, dans lequel le couplage du complexe au trajet d'écoulement comprend la pulvérisation de la solution sur une surface de la bandelette réactive de dosage.
